# EUROPÄISCHE PATENTSCHRIFT

(11) **EP 0 757 680 B1**
(45) Veröffentlichungstag und Bekanntmachung des Hinweises auf die Patenterteilung: **02.07.2003**
(21) Anmeldenummer: 95915200.0
(22) Anmeldetag: 12.04.1995
(51) Int. Cl.: C07D 239/54, A01N 43/54

(54) **N-CYANOARYL-STICKSTOFFHETEROCYCLEN**
N-CYANOARYL NITROGEN HETEROCYCLES
HETEROCYCLES N-CYANOARYLE-AZOTE

(30) Priorität: 25.04.1994 DE 4414326; 19.10.1994 DE 4437295
(43) Veröffentlichungstag der Anmeldung: 12.02.1997
(73) Patentinhaber: Bayer CropScience AG, 40789 Monheim (DE)
(72) Erfinder: ANDREE, Roland, D-40764 Langenfeld (DE); DREWES, Mark, Wilhelm, D-40764 Langenfeld (DE); SANTEL, Hans-Joachim, D-51371 Leverkusen (DE); DOLLINGER, Markus, D-51381 Leverkusen (DE); ERDELEN, Christoph, D-42799 Leichlingen (DE)
(86) Internationale Anmeldenummer: EP9501351
(87) Internationale Veröffentlichungsnummer: WO95029168

(56) Entgegenhaltungen:
- EP-A- 0 408 382
- EP-A- 0 648 749

## Beschreibung

Die Erfindung betrifft N-Cyanoaryl-Stickstoffheterocyclen, mehrere Verfahren zu ihrer Herstellung, ihre Verwendung als Herbizide und Insektizide sowie neue Zwischenprodukte.

Es ist bereits bekannt, dass bestimmte N-Cyanoaryl-Stickstoffheterocyclen herbizide Eigenschaften aufweisen (vgl. WO 91/00278, WO 92/11244, DE 4237920, EP 408382/US 5084084, EP 438209, EP 473551). Die herbizide Wirkung bzw. die Verträglichkeit der vorbekannten N-Cyanoaryl-Stickstoffheterocyclen gegenüber Kulturpflanzen sind jedoch nicht ganz zufriedenstellend.

Es wurden nun die N-Cyanoaryl-Stickstoffheterocyclen der allgemeinen Formel (I) gefunden, 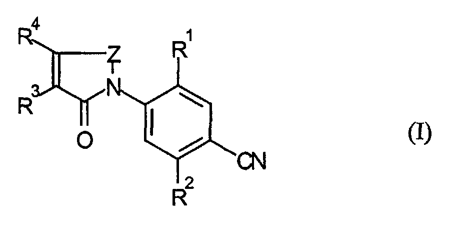 in welcher
- R¹: für Wasserstoff, Fluor, Chlor oder Brom steht,
- R²: für die nachstehende Gruppierung steht, 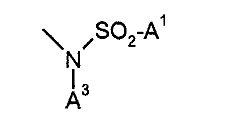 worin
A¹ für gegebenenfalls durch Halogen substituiertes Alkyl mit bis zu 10 Kohlenstoffatomen steht,
A³ für Alkylcarbonyl, Alkoxycarbonyl oder Alkylsulfonyl mit jeweils bis zu 6 Kohlenstoffatomen steht,
- R³: für Wasserstoff, Halogen, Cyano oder für gegebenenfalls durch Halogen substituiertes Alkyl mit 1 bis 6 Kohlenstoffatomen steht,
- R⁴: für gegebenenfalls durch Halogen oder C₁-C₄-Alkoxy substituiertes Alkyl mit 1 bis 6 Kohlenstoffatomen steht,
oder zusammen mit R³ für Alkandiyl mit 2 bis 8 Kohlenstoffatomen steht, und
- Z: für eine der nachstehenden Gruppierungen steht 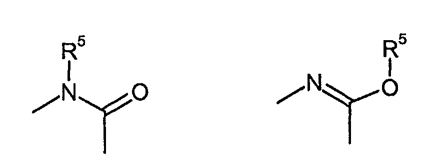 worin
R⁵ für Wasserstoff oder jeweils gegebenenfalls durch Fluor, Chlor, Brom, Cyano, C₁-C₄-Alkoxy, C₁-C₄-Alkyl-carbonyl oder C₁-C₄-Alkoxycarbonyl substituiertes Alkyl, Alkenyl, Alkinyl, Alkylcarbonyl oder Alkoxycarbonyl mit jeweils bis zu 6 Kohlenstoffatomen steht, oder für (jeweils nur an N gebundenes) Amino oder Hydroxy steht.

Die allgemeine Formel (I) steht also für die isomeren Verbindungen der nachstehenden allgemeinen Formeln (IA) und (IB) 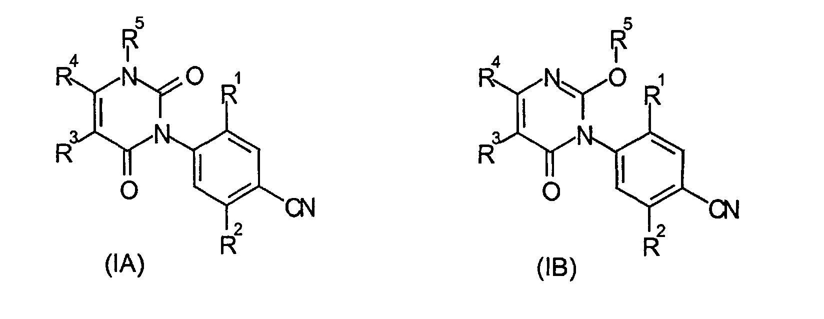 Man erhält die N-Cyanoaryl-Stickstoffheterocyclen der allgemeinen Formel (I), wenn man
(a) zur Herstellung von Verbindungen der Formeln (IA) und (IB), in welchen R⁵ für Wasserstoff steht sowie R¹, R², R³ und R⁴ die oben angegebenen Bedeutungen haben,
   Aminoalkensäureester der allgemeinen Formel (II) 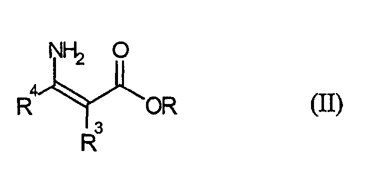 in welcher
   - R³ und R⁴: die oben angegebenen Bedeutungen haben und
   - R: für Alkyl, Aryl oder Arylalkyl steht,
   mit Cyanoarylisocyanaten der allgemeinen Formel (III) 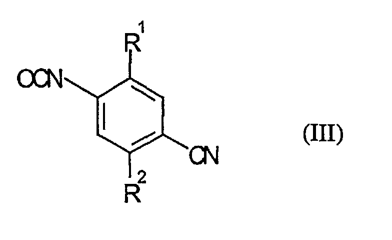 in welcher
   - R¹ und R²: die oben angegebenen Bedeutungen haben,
   gegebenenfalls in Gegenwart eines Reaktionshilfsmittels und gegebenenfalls in Gegenwart eines Verdünnungsmittels umsetzt,
   oder wenn man
(b) zur Herstellung von Verbindungen der Formeln (IA) und/oder (IB), in welchen R⁵ für jeweils gegebenenfalls substituiertes Alkyl, Alkenyl, Alkinyl, Alkylcarbonyl oder Alkoxycarbonyl steht sowie R¹, R², R³ und R⁴ die oben angegebenen Bedeutungen haben,
   N-Cyanoaryl-Stickstoffheterocyclen der allgemeinen Formeln (IA) und/oder (IB)
   in welchen
   - R⁵: für Wasserstoff steht sowie R¹, R², R³ und R⁴ die oben angegebenen Bedeutungen haben,
   mit Alkylierungsmitteln bzw. Acylierungsmitteln der allgemeinen Formeln (V) oder (VI)

   X¹-R⁵ (V)

   R⁵-O-SO₂-O-R⁵ (VI)

   in welchen
   - R⁵: für jeweils gegebenenfalls substituiertes Alkyl, Alkenyl, Alkinyl, Alkylcarbonyl oder Alkoxycarbonyl steht und
   - X¹: in der Formel (V) für Halogen steht,
   gegebenenfalls in Gegenwart eines Säureakzeptors und gegebenenfalls in Gegenwart eines Verdünnungsmittels umsetzt,
   oder wenn man
(c) zur Herstellung von Verbindungen der Formel (I), in welcher R² für die nachstehende Gruppierung steht 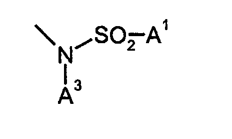 sowie A¹, A³, R¹, R³, R⁴ und Z die oben angegebenen Bedeutungen haben, N-Cyanoaryl-Stickstoffheterocyclen der allgemeinen Formel (I), in welcher R² für die Gruppierung -NH-SO₂-A¹ steht sowie A¹, R¹, R³, R⁴ und Z die oben angegebenen Bedeutungen haben,
   mit Halogenverbindungen der allgemeinen Formel (VII)

   X²-A³ (VII)

   in welcher
   - A³: die oben angegebene Bedeutung hat und
   - X²: für Halogen steht,
   gegebenenfalls in Gegenwart eines Säureakzeptors und gegebenenfalls in Gegenwart eines Verdünnungsmittels umsetzt,
   oder wenn man
(d) zur Herstellung von Verbindungen der Formel (IA), in welcher R⁵ für Amino oder Hydroxy steht sowie R¹, R², R³ und R⁴ die oben angegebenen Bedeutungen haben,
   N-Cyanoaryl-Stickstoffheterocyclen der allgemeinen Formeln (IA) und/oder (IB), in welchen R⁵ für Wasserstoff steht sowie R¹, R², R³ und R⁴ die oben angegebenen Bedeutungen haben,
   mit elektrophilen Aminierungs- bzw. Hydroxylierungsmitteln gegebenenfalls in Gegenwart eines Säureakzeptors und gegebenenfalls in Gegenwart eines Verdünnungsmittels umsetzt.

Die neuen N-Cyanoaryl-Stickstoffheterocyclen der allgemeinen Formel (I) zeichnen sich durch starke herbizide Wirksamkeit aus.

In den Definitionen sind die gesättigten oder ungesättigten Kohlenwasserstoffketten, wie Alkyl, Alkandiyl, Alkenyl oder Alkinyl, jeweils geradkettig oder verzweigt.

Halogen steht im Allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- R¹: für Wasserstoff, Fluor oder Chlor steht,
- R²: für die nachstehende Gruppierung steht, 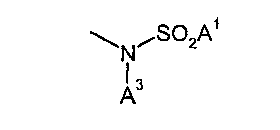 worin
A¹ für gegebenenfalls durch Fluor oder Chlor substituiertes Methyl, Ethyl, n- oder i-Propyl, n-, i-, s- oder t-Butyl, n-, i-, s- oder t-Pentyl steht,
A³ für Acetyl, Propionyl, n- oder i-Butyroyl, Methoxycarbonyl, Ethoxycarbonyl, n- oder i-Propoxycarbonyl, Methylsulfonyl, Ethylsulfonyl, n- oder i-Propylsulfonyl steht,
- R³: für Wasserstoff, Fluor, Chlor, Brom, oder für jeweils gegebenenfalls durch Fluor und/oder Chlor substituiertes Methyl, Ethyl, n- oder i-Propyl steht,
- R⁴: für jeweils gegebenenfalls durch Fluor und/oder Chlor substituiertes Methyl, Ethyl, n- oder i-Propyl steht,
oder zusammen mit R³ für Trimethylen oder Tetramethylen steht, und
- Z: für eine der nachstehenden Gruppierungen steht 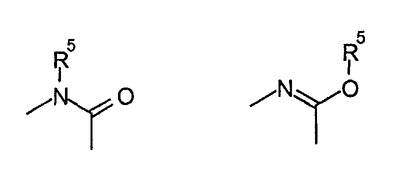 worin
R⁵ für Wasserstoff oder für jeweils gegebenenfalls durch Fluor, Chlor oder Cyano substituiertes Methyl, Ethyl, n- oder i-Propyl, n-, i- oder s-Butyl, Propenyl, Butenyl, Propinyl, Butinyl, Acetyl, Propionyl, Methoxycarbonyl oder Ethoxycarbonyl steht, oder für (jeweils nur an N gebundenes) Amino oder Hydroxy steht.

Eine ganz besonders bevorzugte Gruppe von Verbindungen der Formel (I) sind die Verbindungen der Formel (IA), in welcher R¹, R², R³, R⁴ und R⁵ die oben als insbesondere bevorzugt angegebenen Bedeutungen haben.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Beispiele für die erfindungsgemäßen Verbindungen der Formel (I) bzw. (IA) sind in der nachstehenden Tabelle 1 aufgeführt.

**Tabelle 1:**

| Beispiele für die Verbindungen der Formel (I) bzw. (IA) | | | | |
|---|---|---|---|---|
| R¹ | R² | R³ | R⁴ | R⁵ |
| H | -N(SO₂CH₃)₂ | H | CF₃ | CH₃ |
| H | -N(SO₂CH₃)(SO₂C₂H₅) | H | CF₃ | CH₃ |
| H | -N(SO₂C₂H₅)₂ | H | CF₃ | CH₃ |
| H | -N(SO₂CH₃)(SO₂C₃H₇) | H | CF₃ | CH₃ |
| H | -N(SO₂C₃H₇)₂ | H | CF₃ | CH₃ |
| H | -N(SO₂CH₃)(SO₂C₃H₇-i) | H | CF₃ | CH₃ |
| H | -N(SO₂C₃H₇-i)₂ | H | CF₃ | CH₃ |
| F | -N(SO₂CH₃)₂ | H | CF₃ | CH₃ |
| F | -N(SO₂CH₃)₂ | H | CF₃ | C₂H₅ |
| F | -N(SO₂CH₃)(SO₂C₂H₅) | H | CF₃ | CH₃ |
| F | -N(SO₂C₂H₅)₂ | H | CF₃ | CH₃ |
| F | -N(SO₂CH₃)(SO₂C₃H₇) | H | CF₃ | CH₃ |
| F | -N(SO₂C₃H₇)₂ | H | CF₃ | CH₃ |
| F | -N(SO₂CH₃)(SO₂C₃H₇-i) | H | CF₃ | CH₃ |
| F | -N(SO₂C₃H₇-i)₂ | H | CF₃ | CH₃ |
| F | -N(SO₂C₂H₅)(SO₂C₃H₇) | H | CF₃ | CH₃ |
| F | -N(SO₂CH₃)(SO₂C₄H₉) | H | CF₃ | CH₃ |
| F | -N(SO₂C₄H₉)₂ | H | CF₃ | CH₃ |
| F | -N(SO₂CH₃)(SO₂CF₃) | H | CF₃ | CH₃ |
| F | -N(SO₂CH₃)(SO₂CH₂CF₃) | H | CF₃ | CH₃ |
| F | -N(SO₂CH₃)(SO₂C₃H₆Cl) | H | CF₃ | CH₃ |
| F | -N(SO₂CH₃)₂ | H | CHF₂ | CH₃ |
| F | -N(SO₂CH₃)₂ | H | CHF₂ | C₂H₅ |
| F | -N(SO₂CH₃)(SO₂C₂H₅) | H | CHF₂ | CH₃ |
| F | -N(SO₂C₂H₅)₂ | H | CHF₂ | CH₃ |
| F | -N(SO₂CH₃)(SO₂C₃H₇) | H | CHF₂ | CH₃ |
| F | -N(SO₂C₃H₇)₂ | H | CHF₂ | CH₃ |
| F | -N(SO₂CH₃)(SO₂C₃H₇-i) | H | CHF₂ | CH₃ |
| F | -N(SO₂C₃H₇-i)₂ | H | CHF₂ | CH₃ |
| F | -N(SO₂C₂H₅)(SO₂C₃H₇) | H | CHF₂ | CH₃ |
| F | -N(SO₂CH₃)(SO₂C₄H₉) | H | CHF₂ | CH₃ |
| F | -N(SO₂C₄H₉)₂ | H | CHF₂ | CH₃ |
| F | -N(SO₂CH₃)(SO₂CF₃) | H | CHF₂ | CH₃ |
| F | -N(SO₂CH₃)(SO₂CH₂CF₃) | H | CHF₂ | CH₃ |
| F | -N(SO₂CH₃)(SO₂C₃H₆Cl) | H | CHF₂ | CH₃ |
| F | -N(SO₂CH₃)₂ | H | CF₂Cl | CH₃ |
| F | -N(SO₂CH₃)₂ | H | CF₂Cl | C₂H₅ |
| F | -N(SO₂CH₃)(SO₂C₂H₅) | H | CF₂Cl | CH₃ |
| F | -N(SO₂C₂H₅)₂ | H | CF₂Cl | CH₃ |
| F | -N(SO₂CH₃)(SO₂C₃H₇) | H | CF₂Cl | CH₃ |
| F | -N(SO₂C₃H₇)₂ | H | CF₂Cl | CH₃ |
| F | -N(SO₂CH₃)(SO₂C₃H₇-i) | H | CF₂Cl | CH₃ |
| F | -N(SO₂C₃H₇-i)₂ | H | CF₂Cl | CH₃ |
| F | -N(SO₂C₂H₅)(SO₂C₃H₇) | H | CF₂Cl | CH₃ |
| F | -N(SO₂CH₃)(SO₂C₄H₉) | H | CF₂Cl | CH₃ |
| F | -N(SO₂C₄H₉)₂ | H | CF₂Cl | CH₃ |
| F | -N(SO₂CH₃)(SO₂CF₃) | H | CF₂Cl | CH₃ |
| F | -N(SO₂CH₃)(SO₂CH₂CF₃) | H | CF₂Cl | CH₃ |
| F | -N(SO₂CH₃)(SO₂C₃H₆Cl) | H | CF₂Cl | CH₃ |
| F | -N(SO₂CH₃)₂ | H | C₂F₅ | CH₃ |
| F | -N(SO₂CH₃)₂ | H | C₂F₅ | C₂H₅ |
| F | -N(SO₂CH₃)(SO₂C₂H₅) | H | C₂F₅ | CH₃ |
| F | -N(SO₂C₂H₅)₂ | H | C₂F₅ | CH₃ |
| F | -N(SO₂CH₃)(SO₂C₃H₇) | H | C₂F₅ | CH₃ |
| F | -N(SO₂C₃H₇)₂ | H | C₂F₅ | CH₃ |
| F | -N(SO₂CH₃)(SO₂C₃H₇-i) | H | C₂F₅ | CH₃ |
| F | -N(SO₂C₃H₇-i)₂ | H | C₂F₅ | CH₃ |
| F | -N(SO₂C₂H₅)(SO₂C₃H₇) | H | C₂F₅ | CH₃ |
| F | -N(SO₂CH₃)(SO₂C₄H₉) | H | C₂F₅ | CH₃ |
| F | -N(SO₂C₄H₉)₂ | H | C₂F₅ | CH₃ |
| F | -N(SO₂CH₃)(SO₂CF₃) | H | C₂F₅ | CH₃ |
| F | -N(SO₂CH₃)(SO₂CH₂CF₃) | H | C₂F₅ | CH₃ |
| F | -N(SO₂CH₃)(SO₂C₃H₆Cl) | H | C₂F₅ | CH₃ |
| F | -N(SO₂CH₃)₂ | CH₃ | CF₃ | CH₃ |
| F | -N(SO₂CH₃)₂ | CH₃ | CF₃ | C₂H₅ |
| F | -N(SO₂CH₃)(SO₂C₂H₅) | CH₃ | CF₃ | CH₃ |
| F | -N(SO₂C₂H₅)₂ | CH₃ | CF₃ | CH₃ |
| F | -N(SO₂CH₃)(SO₂C₃H₇) | CH₃ | CF₃ | CH₃ |
| F | -N(SO₂C₃H₇)₂ | CH₃ | CF₃ | CH₃ |
| F | -N(SO₂CH₃)(SO₂C₃H₇-i) | CH₃ | CF₃ | CH₃ |
| F | -N(SO₂C₃H₇-i)₂ | CH₃ | CF₃ | CH₃ |
| F | -N(SO₂C₂H₅)(SO₂C₃H₇) | CH₃ | CF₃ | CH₃ |
| F | -N(SO₂CH₃)(SO₂C₄H₉) | CH₃ | CF₃ | CH₃ |
| F | -N(SO₂C₄H₉)₂ | CH₃ | CF₃ | CH₃ |
| F | -N(SO₂CH₃)(SO₂CF₃) | CH₃ | CF₃ | CH₃ |
| F | -N(SO₂CH₃)(SO₂CH₂CF₃) | CH₃ | CF₃ | CH₃ |
| F | -N(SO₂CH₃)(SO₂C₃H₆Cl) | CH₃ | CF₃ | CH₃ |
| F | -N(SO₂CH₃)₂ | Cl | CF₃ | CH₃ |
| F | -N(SO₂CH₃)₂ | Cl | CF₃ | C₂H₅ |
| F | -N(SO₂CH₃)(SO₂C₂H₅) | Cl | CF₃ | CH₃ |
| F | -N(SO₂C₂H₅)₂ | Cl | CF₃ | CH₃ |
| F | -N(SO₂CH₃)(SO₂C₃H₇) | Cl | CF₃ | CH₃ |
| F | -N(SO₂C₃H₇)₂ | Cl | CF₃ | CH₃ |
| F | -N(SO₂CH₃)(SO₂C₃H₇-i) | Cl | CF₃ | CH₃ |
| F | -N(SO₂C₃H₇-i)₂ | Cl | CF₃ | CH₃ |
| F | -N(SO₂C₂H₅)(SO₂C₃H₇) | Cl | CF₃ | CH₃ |
| F | -N(SO₂CH₃)(SO₂C₄H₉) | Cl | CF₃ | CH₃ |
| F | -N(SO₂C₄H₉)₂ | Cl | CF₃ | CH₃ |
| F | -N(SO₂CH₃)(SO₂CF₃) | Cl | CF₃ | CH₃ |
| F | -N(SO₂CH₃)(SO₂CH₂CF₃) | Cl | CF₃ | CH₃ |
| F | -N(SO₂CH₃)(SO₂C₃H₆Cl) | Cl | CF₃ | CH₃ |
| F | -N(SO₂CH₃)₂ | CH₃ | CHF₂ | CH₃ |
| F | -N(SO₂CH₃)₂ | CH₃ | CHF₂ | C₂H₅ |
| F | -N(SO₂CH₃)(SO₂C₂H₅) | CH₃ | CHF₂ | CH₃ |
| F | -N(SO₂C₂H₅)₂ | CH₃ | CHF₂ | CH₃ |
| F | -N(SO₂CH₃)(SO₂C₃H₇) | CH₃ | CHF₂ | CH₃ |
| F | -N(SO₂C₃H₇)₂ | CH₃ | CHF₂ | CH₃ |
| F | -N(SO₂CH₃)(SO₂C₃H₇-i) | CH₃ | CHF₂ | CH₃ |
| F | -N(SO₂C₃H₇-i)₂ | CH₃ | CHF₂ | CH₃ |
| F | -N(SO₂C₂H₅)(SO₂C₃H₇) | CH₃ | CHF₂ | CH₃ |
| F | -N(SO₂CH₃)(SO₂C₄H₉) | CH₃ | CHF₂ | CH₃ |
| F | -N(SO₂C₄H₉)₂ | CH₃ | CHF₂ | CH₃ |
| F | -N(SO₂CH₃)(SO₂CF₃) | CH₃ | CHF₂ | CH₃ |
| F | -N(SO₂CH₃)(SO₂CH₂CF₃) | CH₃ | CHF₂ | CH₃ |
| F | -N(SO₂CH₃)(SO₂C₃H₆Cl) | CH₃ | CHF₂ | CH₃ |
| F | -N(SO₂CH₃)₂ | Cl | CHF₂ | CH₃ |
| F | -N(SO₂CH₃)₂ | Cl | CHF₂ | C₂H₅ |
| F | -N(SO₂CH₃)(SO₂C₂H₅) | Cl | CHF₂ | CH₃ |
| F | -N(SO₂C₂H₅)₂ | Cl | CHF₂ | CH₃ |
| F | -N(SO₂CH₃)(SO₂C₃H₇) | Cl | CHF₂ | CH₃ |
| F | -N(SO₂C₃H₇)₂ | Cl | CHF₂ | CH₃ |
| F | -N(SO₂CH₃)(SO₂C₃H₇-i) | Cl | CHF₂ | CH₃ |
| F | -N(SO₂C₃H₇-i)₂ | Cl | CHF₂ | CH₃ |
| F | -N(SO₂C₂H₅)(SO₂C₃H₇) | Cl | CHF₂ | CH₃ |
| F | -N(SO₂CH₃)(SO₂C₄H₉) | Cl | CHF₂ | CH₃ |
| F | -N(SO₂C₄H₉)₂ | Cl | CHF₂ | CH₃ |
| F | -N(SO₂CH₃)(SO₂CF₃) | Cl | CHF₂ | CH₃ |
| F | -N(SO₂CH₃)(SO₂CH₂CF₃) | Cl | CHF₂ | CH₃ |
| F | -N(SO₂CH₃)(SO₂C₃H₆Cl) | Cl | CHF₂ | CH₃ |
| H | -N(SO₂CH₃)₂ | H | CF₂Cl | CH₃ |
| H | -N(SO₂CH₃)₂ | H | CF₂Cl | C₂H₅ |
| H | -N(SO₂CH₃)(SO₂C₂H₅) | H | CF₂Cl | CH₃ |
| H | -N(SO₂C₂H₅)₂ | H | CF₂Cl | CH₃ |
| H | -N(SO₂CH₃)(SO₂C₃H₇) | H | CF₂Cl | CH₃ |
| H | -N(SO₂C₃H₇)₂ | H | CF₂Cl | CH₃ |
| H | -N(SO₂CH₃)(SO₂C₃H₇-i) | H | CF₂Cl | CH₃ |
| H | -N(SO₂C₃H₇-i)₂ | H | CF₂Cl | CH₃ |
| H | -N(SO₂C₂H₅)(SO₂C₃H₇) | H | CF₂Cl | CH₃ |
| H | -N(SO₂CH₃)(SO₂C₄H₉) | H | CF₂Cl | CH₃ |
| H | -N(SO₂C₄H₉)₂ | H | CF₂Cl | CH₃ |
| H | -N(SO₂CH₃)(SO₂CF₃) | H | CF₂Cl | CH₃ |
| H | -N(SO₂CH₃)(SO₂CH₂CF₃) | H | CF₂Cl | CH₃ |
| H | -N(SO₂CH₃)(SO₂C₃H₆Cl) | H | CF₂Cl | CH₃ |
| F | -N(SO₂CH₃)₂ | CH₃ | CF₂Cl | CH₃ |
| F | -N(SO₂CH₃)₂ | CH₃ | CF₂Cl | C₂H₅ |
| F | -N(SO₂CH₃)(SO₂C₂H₅) | CH₃ | CF₂Cl | CH₃ |
| F | -N(SO₂C₂H₅)₂ | CH₃ | CF₂Cl | CH₃ |
| F | -N(SO₂CH₃)(SO₂C₃H₇) | CH₃ | CF₂Cl | CH₃ |
| F | -N(SO₂C₃H₇)₂ | CH₃ | CF₂Cl | CH₃ |
| F | -N(SO₂CH₃)(SO₂C₃H₇-i) | CH₃ | CF₂Cl | CH₃ |
| F | -N(SO₂C₃H₇-i)₂ | CH₃ | CF₂Cl | CH₃ |
| F | -N(SO₂C₂H₅)(SO₂C₃H₇) | CH₃ | CF₂Cl | CH₃ |
| F | -N(SO₂CH₃)(SO₂C₄H₉) | CH₃ | CF₂Cl | CH₃ |
| F | -N(SO₂C₄H₉)₂ | CH₃ | CF₂Cl | CH₃ |
| F | -N(SO₂CH₃)(SO₂CF₃) | CH₃ | CF₂Cl | CH₃ |
| F | -N(SO₂CH₃)(SO₂CH₂CF₃) | CH₃ | CF₂Cl | CH₃ |
| F | -N(SO₂CH₃)(SO₂C₃H₆Cl) | CH₃ | CF₂Cl | CH₃ |
| F | -N(SO₂CH₃)₂ | Cl | CF₂Cl | CH₃ |
| F | -N(SO₂CH₃)₂ | Cl | CF₂Cl | C₂H₅ |
| F | -N(SO₂CH₃)(SO₂C₂H₅) | Cl | CF₂Cl | CH₃ |
| F | -N(SO₂C₂H₅)₂ | Cl | CF₂Cl | CH₃ |
| F | -N(SO₂CH₃)(SO₂C₃H₇) | Cl | CF₂Cl | CH₃ |
| F | -N(SO₂C₃H₇)₂ | Cl | CF₂Cl | CH₃ |
| F | -N(SO₂CH₃)(SO₂C₃H₇-i) | Cl | CF₂Cl | CH₃ |
| F | -N(SO₂C₃H₇-i)₂ | Cl | CF₂Cl | CH₃ |
| F | -N(SO₂C₂H₅)(SO₂C₃H₇) | Cl | CF₂Cl | CH₃ |
| F | -N(SO₂CH₃)(SO₂C₄H₉) | Cl | CF₂Cl | CH₃ |
| F | -N(SO₂C₄H₉)₂ | Cl | CF₂Cl | CH₃ |
| F | -N(SO₂CH₃)(SO₂CF₃) | Cl | CF₂Cl | CH₃ |
| F | -N(SO₂CH₃)(SO₂CH₂CF₃) | Cl | CF₂Cl | CH₃ |
| F | -N(SO₂CH₃)(SO₂C₃H₆Cl) | Cl | CF₂Cl | CH₃ |
| F | -N(SO₂CH₃)₂ | H | CF₃ | C₂H₅ |
| F | -N(SO₂CH₃)(SO₂C₂H₅) | H | CF₃ | C₂H₅ |
| F | -N(SO₂C₂H₅)₂ | H | CF₃ | C₂H₅ |
| F | -N(SO₂CH₃)(SO₂C₃H₇) | H | CF₃ | C₂H₅ |
| F | -N(SO₂C₃H₇)₂ | H | CF₃ | C₂H₅ |
| F | -N(SO₂CH₃)(SO₂C₃H₇-i) | H | CF₃ | C₂H₅ |
| F | -N(SO₂C₃H₇-i)₂ | H | CF₃ | C₂H₅ |
| F | -N(SO₂C₂H₅)(SO₂C₃H₇) | H | CF₃ | C₂H₅ |
| F | -N(SO₂CH₃)(SO₂C₄H₉) | H | CF₃ | C₂H₅ |
| F | -N(SO₂C₄H₉)₂ | H | CF₃ | C₂H₅ |
| F | -N(SO₂CH₃)(SO₂CF₃) | H | CF₃ | C₂H₅ |
| F | -N(SO₂CH₃)(SO₂CH₂CF₃) | H | CF₃ | C₂H₅ |
| F | -N(SO₂CH₃)(SO₂C₃H₆Cl) | H | CF₃ | C₂H₅ |
| F | -N(SO₂CH₃)(SO₂C₂H₅) | H | CF₂Cl | C₂H₅ |
| F | -N(SO₂CH₃)(SO₂C₄H₉) | H | CF₂Cl | C₂H₅ |
| F | -N(SO₂C₄H₉)₂ | H | CF₂Cl | C₂H₅ |
| F | -N(SO₂CH₃)(SO₂CF₃) | H | CF₂Cl | C₂H₅ |
| F | -N(SO₂CH₃)(SO₂CH₂CF₃) | H | CF₂Cl | C₂H₅ |
| F | -N(SO₂CH₃)(SO₂C₃H₆Cl) | H | CF₂Cl | C₂H₅ |
| F | -N(SO₂CH₃)(COOCH₃) | H | CF₃ | CH₃ |
| F | -N(SO₂CH₃)(COOC₂H₅) | H | CF₃ | CH₃ |
| F | -N(SO₂CH₃)(COOC₄H₉-t) | H | CF₃ | CH₃ |
| F | -N(SO₂CH₃)(COCH₃) | H | CF₃ | CH₃ |
| F | -N(SO₂C₂H₅)(COOCH₃) | H | CF₃ | CH₃ |
| F | -N(SO₂C₂H₅)(COOC₂H₅) | H | CF₃ | CH₃ |
| F | -N(SO₂C₂H₅)(COOC₄H₉-t) | H | CF₃ | CH₃ |
| F | -N(SO₂C₂H₅)(COCH₃) | H | CF₃ | CH₃ |
| F | -N(SO₂C₃H₇)(COOCH₃) | H | CF₃ | CH₃ |
| F | -N(SO₂C₃H₇)(COOC₂H₅) | H | CF₃ | CH₃ |
| F | -N(SO₂C₃H₇)(COOC₄H₉-t) | H | CF₃ | CH₃ |
| F | -N(SO₂C₃H₇)(COCH₃) | H | CF₃ | CH₃ |
| F | -N(SO₂C₄H₉)(COOCH₃) | H | CF₃ | CH₃ |
| F | -N(SO₂C₄H₉)(COOC₂H₅) | H | CF₃ | CH₃ |
| F | -N(SO₂C₄H₉)(COOC₄H₉-t) | H | CF₃ | CH₃ |
| F | -N(SO₂C₄H₉)(COCH₃) | H | CF₃ | CH₃ |
| F | -N(SO₂CH₂CF₃)(COOCH₃) | H | CF₃ | CH₃ |
| F | -N(SO₂CH₂CF₃)(COOC₂H₅) | H | CF₃ | CH₃ |
| F | -N(SO₂CH₂CF₃)(COOC₄H₉-t) | H | CF₃ | CH₃ |
| F | -N(SO₂CH₂CF₃)(COCH₃) | H | CF₃ | CH₃ |
| F | -N(SO₂CH₃)(COOCH₃) | H | CHF₂ | CH₃ |
| F | -N(SO₂C₂H₅)(COOCH₃) | H | CHF₂ | CH₃ |
| F | -N(SO₂CH₃)(COOC₂H₅) | H | CHF₂ | CH₃ |
| -F | -N(SO₂CH₃)(COOCH₃) | H | CClF₂ | CH₃ |
| F | -N(SO₂C₂H₅)(COOCH₃) | H | CClF₂ | CH₃ |
| F | -N(SO₂CH₃)(COOC₂H₅) | H | CClF₂ | CH₃ |
| H | -N(SO₂CH₃)₂ | H | CF₃ | NH₂ |
| H | -N(SO₂CH₃)(SO₂C₂H₅) | H | CF₃ | NH₂ |
| H | -N(SO₂C₂H₅)₂ | H | CF₃ | NH₂ |
| H | -N(SO₂CH₃)(SO₂C₃H₇) | H | CF₃ | NH₂ |
| H | -N(SO₂C₃H₇)₂ | H | CF₃ | NH₂ |
| H | -N(SO₂CH₃)(SO₂C₃H₇-i) | H | CF₃ | NH₂ |
| H | -N(SO₂C₃H₇-i)₂ | H | CF₃ | NH₂ |
| F | -N(SO₂CH₃)₂ | H | CF₃ | NH₂ |
| F | -N(SO₂CH₃)(SO₂C₂H₅) | H | CF₃ | NH₂ |
| F | -N(SO₂C₂H₅)₂ | H | CF₃ | NH₂ |
| F | -N(SO₂CH₃)(SO₂C₃H₇) | H | CF₃ | NH₂ |
| F | -N(SO₂C₃H₇)₂ | H | CF₃ | NH₂ |
| F | -N(SO₂CH₃)(SO₂C₃H₇-i) | H | CF₃ | NH₂ |
| F | -N(SO₂C₃H₇-i)₂ | H | CF₃ | NH₂ |
| F | -N(SO₂C₂H₅)(SO₂C₃H₇) | H | CF₃ | NH₂ |
| F | -N(SO₂CH₃)(SO₂C₄H₉) | H | CF₃ | NH₂ |
| F | -N(SO₂C₄H₉)₂ | H | CF₃ | NH₂ |
| F | -N(SO₂CH₃)(SO₂CF₃) | H | CF₃ | NH₂ |
| F | -N(SO₂CH₃)(SO₂CH₂CF₃) | H | CF₃ | NH₂ |
| F | -N(SO₂CH₃)₂ | H | CHF₂ | NH₂ |
| F | -N(SO₂CH₃)(SO₂C₂H₅) | H | CHF₂ | NH₂ |
| F | -N(SO₂C₂H₅)₂ | H | CHF₂ | NH₂ |
| F | -N(SO₂CH₃)(SO₂C₃H₇) | H | CHF₂ | NH₂ |
| F | -N(SO₂C₃H₇)₂ | H | CHF₂ | NH₂ |
| F | -N(SO₂CH₃)(SO₂C₃H₇-i) | H | CHF₂ | NH₂ |
| F | -N(SO₂C₃H₇-i)₂ | H | CHF₂ | NH₂ |
| F | -N(SO₂C₂H₅)(SO₂C₃H₇) | H | CHF₂ | NH₂ |
| F | -N(SO₂CH₃)₂ | H | CF₂Cl | NH₂ |
| F | -N(SO₂CH₃)(SO₂C₂H₅) | H | CF₂Cl | NH₂ |
| F | -N(SO₂C₂H₅)₂ | H | CF₂Cl | NH₂ |
| F | -N(SO₂CH₃)(SO₂C₃H₇) | H | CF₂Cl | NH₂ |
| F | -N(SO₂C₃H₇)₂ | H | CF₂Cl | NH₂ |
| F | -N(SO₂CH₃)(SO₂C₃H₇-i) | H | CF₂Cl | NH₂ |
| F | -N(SO₂C₃H₇-i)₂ | H | CF₂Cl | NH₂ |
| F | -N(SO₂C₂H₅)(SO₂C₃H₇) | H | CF₂Cl | NH₂ |
| F | -N(SO₂CH₃)(SO₂C₂H₅) | H | C₂F₅ | NH₂ |
| F | -N(SO₂C₂H₅)₂ | H | C₂F₅ | NH₂ |
| F | -N(SO₂CH₃)(SO₂C₃H₇) | H | C₂F₅ | NH₂ |
| F | -N(SO₂C₃H₇)₂ | H | C₂F₅ | NH₂ |
| F | -N(SO₂CH₃)(SO₂C₃H₇-i) | H | C₂F₅ | NH₂ |
| F | -N(SO₂C₃H₇-i)₂ | H | C₂F₅ | NH₂ |
| F | -N(SO₂C₂H₅)(SO₂C₃H₇) | H | C₂F₅ | NH₂ |
| Cl | -N(SO₂CH₃)₂ | H | CF₃ | NH₂ |
| Cl | -N(SO₂CH₃)(SO₂C₂H₅) | H | CF₃ | NH₂ |
| Cl | -N(SO₂CH₃)₂ | F | CF₃ | NH₂ |
| Cl | -N(SO₂CH₃)(SO₂C₂H₅) | F | CF₃ | NH₂ |
| F | -N(SO₂CH₃)₂ | Cl | CF₃ | NH₂ |
| F | -N(SO₂CH₃)(SO₂C₂H₅) | Cl | CF₃ | NH₂ |
| F | -N(SO₂CH₃)₂ | CH₃ | CF₃ | NH₂ |
| F | -N(SO₂CH₃)(SO₂C₂H₅) | CH₃ | CF₃ | NH₂ |
| F | -N(SO₂CH₃)₂ | H | CF₃ | OH |
| F | -N(SO₂CH₃)(SO₂C₂H₅) | H | CHF₂ | OH |
| F | -N(SO₂CH₃)₂ | H | CF₂Cl | OH |
| F | -N(SO₂CH₃)(SO₂C₂H₅) | H | C₂F₅ | OH |

Verwendet man beispielsweise 3-Amino-crotonsäure-methylester und N-Methoxycarbonyl-N-(2-cyano-4-fluor-5-isocyanato-phenyl)-methansulfonsäureamid als Ausgangsstoffe, so kann der Reaktionsablauf beim erfindungsgemäßen Verfahren (a) durch das folgende Formelschema skizziert werden:

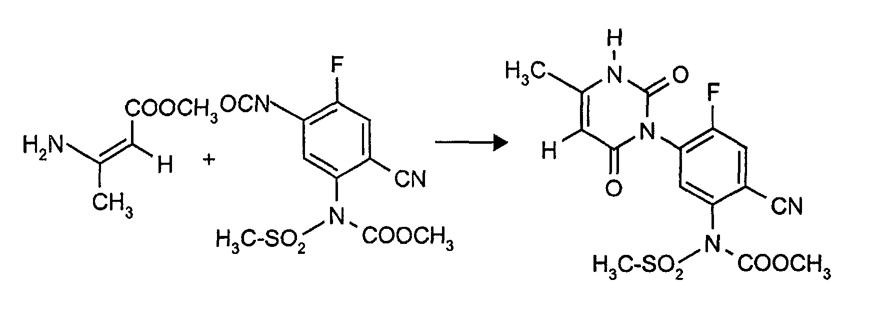

Verwendet man beispielsweise 1-[2-Chlor-4-cyano-5-(N,N-bis-methylsulfonyl)-amino-phenyl]-3,6-dihydro-2,6-dioxo-4-trifluormethyl-1(2H)-pyrimidin und Methylbromid als Ausgangsstoffe, so kann der Reaktionsablauf beim erfindungsgemäßen Verfahren (b) durch das folgende Formelschema skizziert werden:

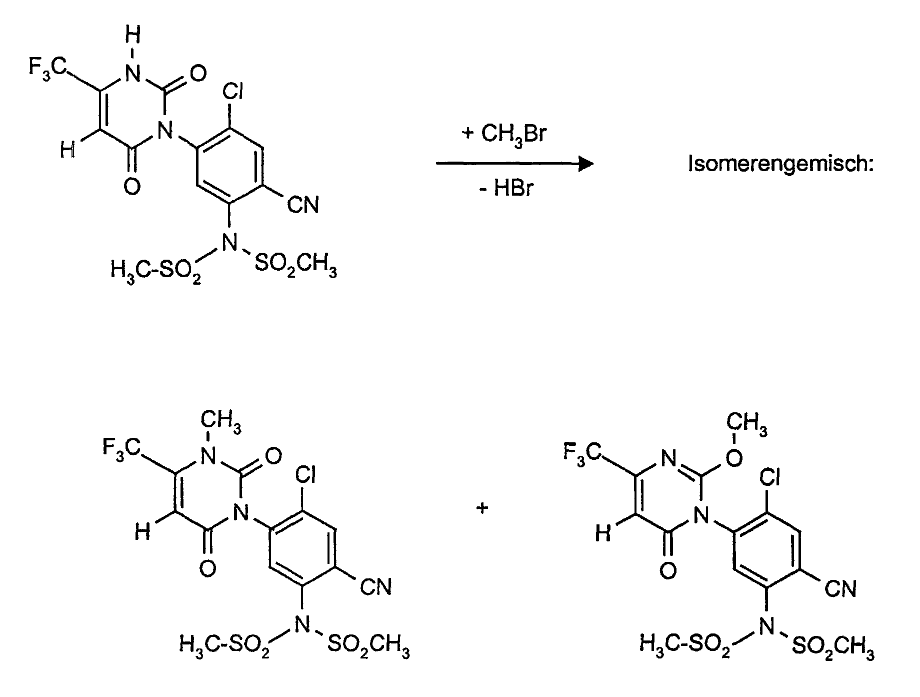

Verwendet man beispielsweise 1-(4-Cyano-2-fluor-5-ethylsulfonylamino-phenyl)-3,6-dihydro-3,4-dimethyl-2,6-dioxo-1(2H)-pyrimidin und Methansulfonsäurechlorid als Ausgangsstoffe, so kann der Reaktionsablauf beim erfindungsgemäßen Verfahren (c) durch das folgende Formelschema skizziert werden:

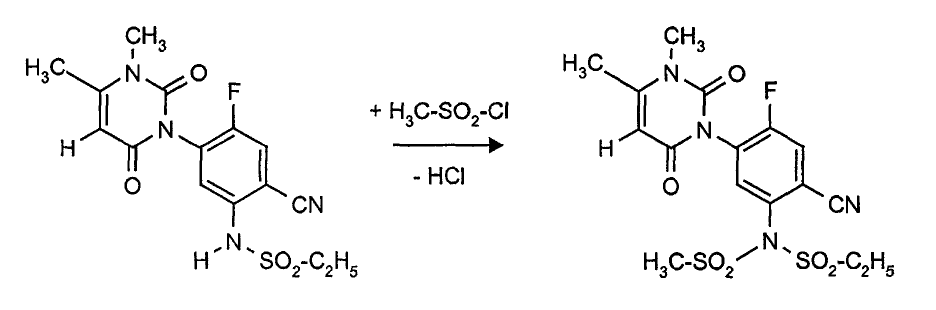

Verwendet man beispielsweise 1-[2-Fluor-4-cyano-5-(N,N-bis-methylsulfonyl)-amino-phenyl]-3,6-dihydro-2,6-dioxo-4-trifluormethyl-1(2H)-pyrimidin und 1-Aminooxy-2,4-dinitro-benzol (ADNB) als Ausgangsstoffe, so kann der Reaktionsablauf beim erfindungsgemäßen Verfahren (d) durch das folgende Formelschema skizziert werden:

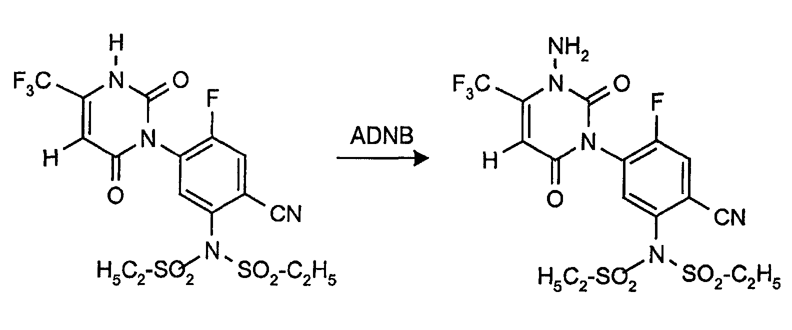

Die beim erfindungsgemäßen Verfahren (a) zur Herstellung von Verbindungen der Formel (I) als Ausgangsstoffe zu verwendenden Aminoalkensäureester sind durch die Formel (II) allgemein definiert. In der Formel (II) haben R³ und R⁴ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R³ und R⁴ angegeben wurden; R steht vorzugsweise für C₁-C₄-Alkyl, Phenyl oder Benzyl, insbesondere für Methyl oder Phenyl.

Die Ausgangsstoffe der Formel (II) sind bekannt und/oder können nach an sich bekannten Verfahren hergestellt werden (vgl. J. Heterocycl. Chem. 9 (1972), 513-522).

Die beim erfindungsgemäßen Verfahren (a) weiter als Ausgangsstoffe zu verwendenden Cyanoarylisocyanate sind durch die Formel (III) allgemein definiert. In der Formel (III) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden.

Die Cyanoarylisocyanate der allgemeinen Formel (III) sind noch nicht aus der Literatur bekannt; sie sind jedoch Gegenstand einer vorgängigen Anmeldung (vgl. DE 4 327 743).

Man erhält die Cyanoarylisocyanate der Formel (III), wenn man Cyanoarylamine der allgemeinen Formel (VIII) 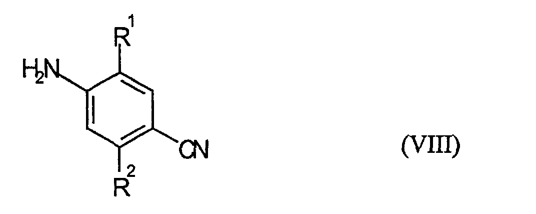 in welcher
- R¹ und R²: die oben angegebenen Bedeutungen haben,
mit Phosgen in Gegenwart eines Verdünnungsmittels, wie z.B. Chlorbenzol, bei Temperaturen zwischen -20°C und +150°C umsetzt (vgl. die Herstellungsbeispiele).

Das erfindungsgemäße Verfahren (a) zur Herstellung der N-Cyanoaryl-Stickstoffheterocyclen der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol, Ether wie Diethyl- und Dibutyl-ether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigsäure-methylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Das erfindungsgemäße Verfahren (a) wird vorzugsweise in Gegenwart eines Reaktionshilfsmittels durchgeführt. Als Reaktionshilfsmittel sind hierbei vor allem Säureakzeptoren geeignet. Vorzugsweise infrage kommen Alkalimetall- und Erdalkalimetall-hydride, wie Lithium-, Natrium-, Kalium- und Calcium-hydrid, Alkalimetall- und Erdalkalimetall-hydroxide, wie Lithium-, Natrium-, Kalium- und Calcium-hydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im Allgemeinen arbeitet man bei Temperaturen zwischen -120°C und +100°C, vorzugsweise bei Temperaturen zwischen -70°C und +80°C.

Das erfindungsgemäße Verfahren (a) wird im Allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im Allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im Allgemeinen in einem geeigneten Verdünnungsmittel in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die beim erfindungsgemäßen Verfahren (b) zur Herstellung von Verbindungen der Formel (I) als Ausgangsstoffe zu verwendenden N-Cyanoaryl-Stickstoffheterocyclen sind durch die Formeln (IA) und (IB) - mit der Maßgabe, dass darin R⁵ für Wasserstoff steht - allgemein definiert. In den Formeln (IA) und (IB) haben R¹, R², R³ und R⁴ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹, R², R³ und R⁴ angegeben wurden.

Die Ausgangsstoffe der Formeln (IA) und (IB) für Verfahren (b) sind erfmdungsgemäße neue Verbindungen; sie können nach dem erfindungsgemäßen Verfahren (a) hergestellt werden.

Die beim erfindungsgemäßen Verfahren (b) weiter als Ausgangsstoffe zu verwendenden Alkylierungsmittel sind durch die Formeln (IV) und (V) allgemein definiert. In den Formeln (IV) und (V) hat R⁵ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R⁵ angegeben wurde.

Die Ausgangsstoffe der Formeln (IV) und (V) sind bekannte organische Synthesechemikalien.

Das erfindungsgemäße Verfahren (b) wird vorzugsweise unter Verwendung eines Verdünnungsmittels durchgeführt. Es kommen hierbei vor allem diejenigen Verdünnungsmittel in Betracht, die bereits bei der Beschreibung des erfindungsgemäßen Verfahrens (a) genannt wurden.

Als Säureakzeptoren können bei dem erfindungsgemäßen Verfahren (b) alle üblicherweise für derartige Umsetzungen verwendbaren Säurebindemittel eingesetzt werden. Vorzugsweise infrage kommen Alkalimetall- und Erdalkalimetall-hydride, wie Lithium-, Natrium-, Kalium- und Calcium-hydrid, Alkalimetall- und Erdalkalimetall-hydroxide, wie Lithium-, Natrium-, Kalium- und Calcium-hydroxid, Alkalimetallund Erdalkalimetall-carbonate und -hydrogencarbonate, wie Natrium- und Kaliumcarbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (b) in einem größeren Bereich variiert werden. Im Allgemeinen arbeitet man bei Temperaturen zwischen 0°C und 120°C, vorzugsweise bei Temperaturen zwischen 10°C und 100°C.

Das erfindungsgemäße Verfahren (b) wird im Allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im Allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im Allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden (vgl. die Herstellungsbeispiele).

Die beim erfindungsgemäßen Verfahren (c) zur Herstellung von Verbindungen der Formel (I) als Ausgangsstoffe zu verwendenden N-Cyanoaryl-Stickstoffheterocyclen sind durch die Formel (I) - mit der Maßgabe, dass darin R² für die Gruppierung -NH-SO₂-A¹ steht - allgemein definiert. In der Formel (I) haben dann A¹, R¹, R³, R⁴ und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A¹, R¹, R³, R⁴ und Z angegeben wurden.

Die Ausgangsstoffe der Formel (I) für Verfahren (c) sind Gegenstand vorgängiger Anmeldungen (vgl. DE 4 327 743; vgl. auch US 5 084 084; Herstellungsbeispiele).

Die beim erfindungsgemäßen Verfahren (c) weiter als Ausgangsstoffe zu verwendenden Halogenverbindungen sind durch die Formel (VII) allgemein definiert. In der Formel (VII) hat A³ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für A³ angegeben wurde; X² steht vorzugsweise für Fluor, Chlor oder Brom, insbesondere für Chlor.

Die Ausgangsstoffe der Formel (VI) sind bekannte organische Synthesechemikalien. Das erfindungsgemäße Verfahren (c) wird vorzugsweise unter Verwendung eines Verdünnungsmittels durchgeführt. Es kommen hierbei vor allem diejenigen Verdünnungsmittels in Betracht, die bereits bei der Beschreibung des erfindungsgemäßen Verfahrens (a) genannt wurden.

Das erfindungsgemäße Verfahren (c) wird gegebenenfalls in Gegenwart eines Säureakzeptors durchgeführt. Es kommen hierbei diejenigen Säureakzeptoren in Betracht, die bereits bei der Beschreibung des erfindungsgemäßen Verfahrens (b) genannt wurden.

Die Reaktionstemperaturen können bei dem erfmdungsgemäßen Verfahren (c) in einem größeren Bereich variiert werden. Im Allgemeinen arbeitet man bei Temperaturen zwischen 0°C und 150°C, vorzugsweise bei Temperaturen zwischen 20°C und 120°C.

Das erfindungsgemäße Verfahren (c) wird im Allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im Allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im Allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden (vgl. die Herstellungsbeispiele).

Die beim erfindungsgemäßen Verfahren (d) zur Herstellung von Verbindungen der Formel (I) als Ausgangsstoffe zu verwendenden N-Cyanoaryl-Stickstoffheterocyclen sind durch die Formeln (IA) und (IB) - mit der Maßgabe, dass darin R⁵ für Wasserstoff steht - allgemein definiert. In den Formeln (IA) und (IB) haben R¹, R², R³ und R⁴ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹, R², R³ und R⁴ angegeben wurden.

Die Ausgangsstoffe der Formeln (IA) und (IB) für Verfahren (c) sind erfindungsgemäße neue Verbindungen; sie können nach dem erfindungsgemäßen Verfahren (a) hergestellt werden.

Das erfindungsgemäße Verfahren (d) wird unter Verwendung eines elektrophilen Aminierungs- bzw. Hydroxylierungsmittels durchgeführt. Es können hierbei die üblichen Aminierungs- bzw. Hydroxylierungsmittel eingesetzt werden. Als Beispiele seien 1-Aminooxy-2,4-dinitro-benzol, Hydroxylamin-O-sulfonsäure, N-(Dialkoxyphosphoryl)-O-(4-Nitro-phenyl-sulfonyl)-hydroxylamin und 3-Chlor-perbenzoesäure genannt. Es handelt sich hierbei um bekannte Synthesechemikalien.

Das erfindungsgemäße Verfahren (d) wird vorzugsweise unter Verwendung eines Verdünnungsmittels durchgeführt. Es kommen hierbei vor allem diejenigen Verdünnungsmittels in Betracht, die bereits bei der Beschreibung des erfindungsgemäßen Verfahrens (a) genannt wurden.

Das erfindungsgemäße Verfahren (d) wird gegebenenfalls in Gegenwart eines Säureakzeptors durchgeführt. Es kommen hierbei diejenigen Säureakzeptoren in Betracht, die bereits bei der Beschreibung des erfindungsgemäßen Verfahrens (b) genannt wurden.

Die Reaktionstemperaturen können bei dem erfindungsgemäßen Verfahren (d) in einem größeren Bereich variiert werden. Im Allgemeinen arbeitet man bei Temperaturen zwischen 0°C und 150°C, vorzugsweise bei Temperaturen zwischen 10°C und 80°C.

Das erfindungsgemäße Verfahren (d) wird im Allgemeinen unter Normaldruck durchgeführt. Es ist jedoch auch möglich, unter erhöhtem oder vermindertem Druck zu arbeiten.

Zur Durchführung des erfindungsgemäßen Verfahrens (d) werden die jeweils benötigten Ausgangsstoffe im Allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im Allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (d) jeweils nach üblichen Methoden (vgl. die Herstellungsbeispiele).

Die erfindungsgemäßen N-Cyanoaryl-Stickstoffheterocyclen können zur Herstellung von Verbindungen der Formel (I), in welcher R² für die Gruppierung -NH-SO₂-A¹ steht, eingesetzt werden.

Man erhält die Verbindungen der Formel (I), in welcher R² für die Gruppierung -NH-SO₂-A¹ steht, wenn man entsprechende Verbindungen der Formel (I), in welcher R¹, R², R³, R⁴ und Z die oben angegebene Bedeutung haben, mit Wasser in Gegenwart eines Reaktionshilfsmittels, wie z.B. Natrium- oder Kalium-acetat, Natrium- oder Kalium-hydrogencarbonat, Natrium-, Kalium- oder Calcium-carbonat, vorzugsweise Natriumhydrogencarbonat, und gegebenenfalls in Gegenwart eines organischen Lösungsmittels, wie z.B. Methanol, Ethanol, n- oder i-Propanol, Aceton, Methylethylketon oder Methylisobutylketon, vorzugsweise Aceton, bei Temperaturen zwischen 0°C und 100°C, vorzugsweise zwischen 10°C und 80°C umsetzt und anschließend mit einer starken Säure, wie z.B. Salzsäure, ansäuert (vgl. die Herstellungsbeispiele).

Die so erhältlichen Verbindungen der Formel (I) sind bereits bekannt (vgl. US 5 084 084) bzw. Gegenstand einer vorgängigen Anmeldung (vgl. DE 4 327 743).

Überraschenderweise können die Verbindungen der Formel (I), in welcher R² für die Gruppierung -NH-SO₂-A¹ steht, nach dem vorausgehend beschriebenen Verfahren in erheblich besserer Ausbeute und Qualität als nach vorbekannten Methoden erhalten werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfmdungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen und dikotylen Kulturen, wie z.B. in Weizen, Mais und Soja, sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Aryl-sulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylhamstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor, als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

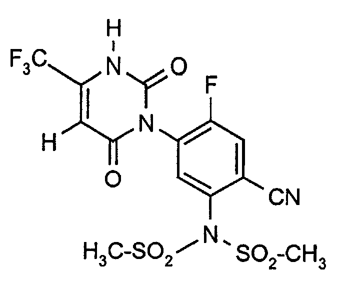

0,63 g (5 mMol) Methansulfonsäurechlorid werden bei 20°C zu einer Mischung aus 1,96 g (5 mMol) 1-(4-Cyano-2-fluor-5-methylsulfonylamino-phenyl)-3,6-dihydro-2,6-dioxo-4-trifluormethyl-1(2H)-pyrimidin, 0,61 g (6 mMol) Triethylamin und 20 ml Acetonitril unter Rühren tropfenweise gegeben. Das Reaktionsgemisch wird 60 Minuten bei 20°C gerührt, mit weiteren 0,6 g Triethylamin und 0,5 g Methansulfonsäurechlorid versetzt und weitere 2 Stunden bei 20°C gerührt. Dann wird eingeengt, der Rückstand mit Wasser/Essigsäureethylester geschüttelt, die organische Phase abgetrennt, mit Natriumsulfat getrocknet und filtriert. Das Filtrat wird eingeengt, der Rückstand mit Diethylether digeriert und das kristallin anfallende Produkt durch Abfiltrieren isoliert.

Man erhält 2,1 g (90 % der Theorie) 1-[4-Cyano-2-fluor-5-(bis-methylsulfonyl-amino)-phenyl]-3,6-dihydro-2,6-dioxo-4-trifluormethyl-1(2H)-pyrimidin vom Schmelzpunkt 143°C.

### Beispiel 2

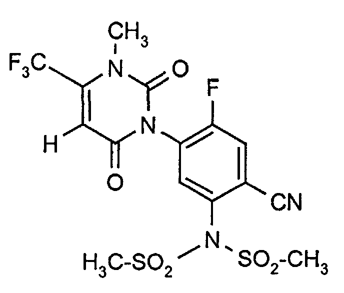

Eine Mischung aus 2,06 g (4,25 mMol) 1-[4-Cyano-2-fluor-5-(bis-methylsulfonylamino)-phenyl]-3,6-dihydro-2,6-dioxo-4-trifluormethyl-1(2H)-pyrimidin, 0,63 g (5,0 mMol) Dimethylsulfat, 0,70 g (5,0 mMol) Kaliumcarbonat und 50 ml Aceton wird 2 Stunden unter Rückfluß erhitzt und anschließend eingeengt. Der Rückstand wird mit Wasser digeriert und das kristallin anfallende Produkt durch Abfiltrieren isoliert.

Man erhält 1,8 g (87% der Theorie) 1-[4-Cyano-2-fluor-5-(bis-methylsulfonylamino)-phenyl]-3,6-dihydro-2,6-dioxo-3-methyl-4-trifluormethyl-1 (2H)-pyrimidin vom Schmelzpunkt 273°C.

Analog zu den Beispielen 1 und 2 sowie entsprechend der allgemeinen Beschreibung der erfindungsgemäßen Herstellungsverfahren können beispielsweise die in der nachstehenden Tabelle 2 aufgeführten Verbindungen der Formel (I) - bzw. der Formeln (IA) und (IB) - hergestellt werden:

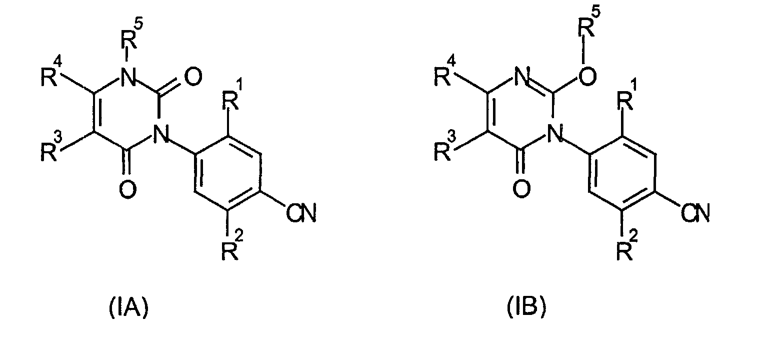

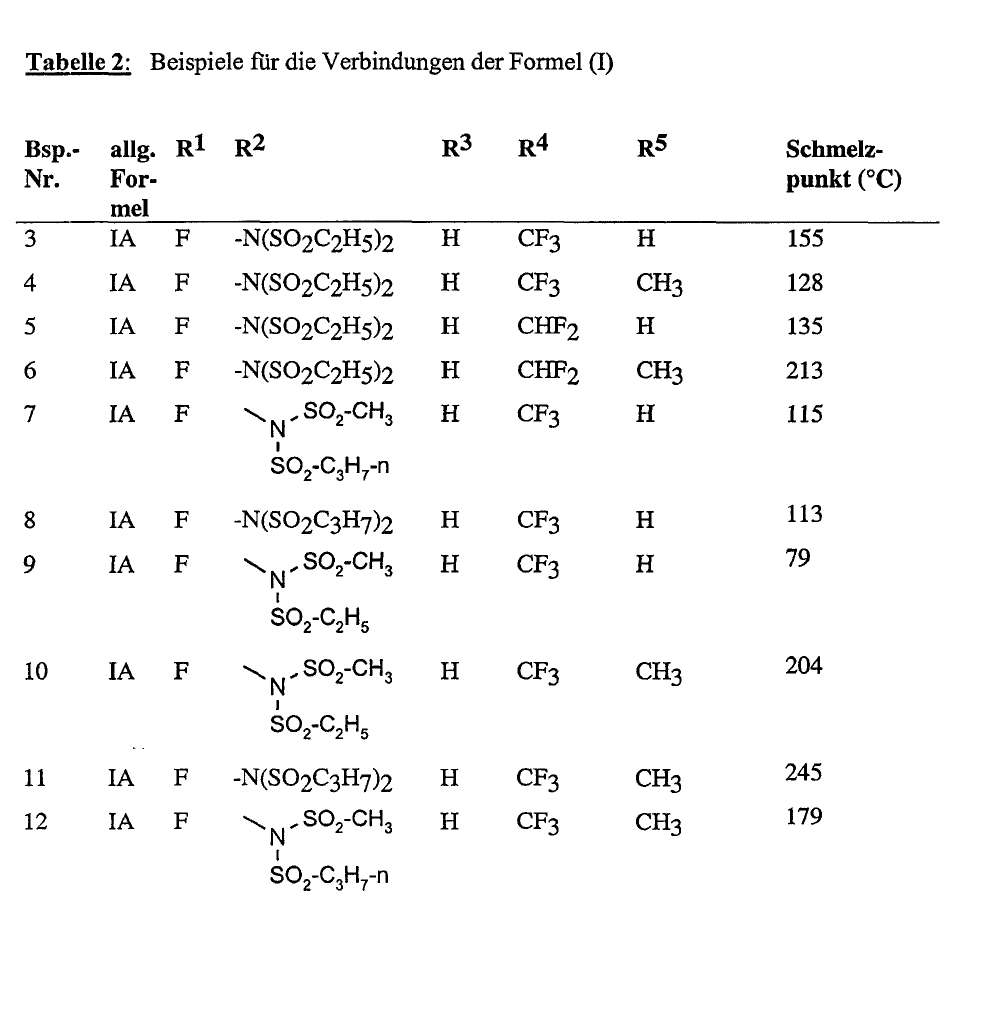

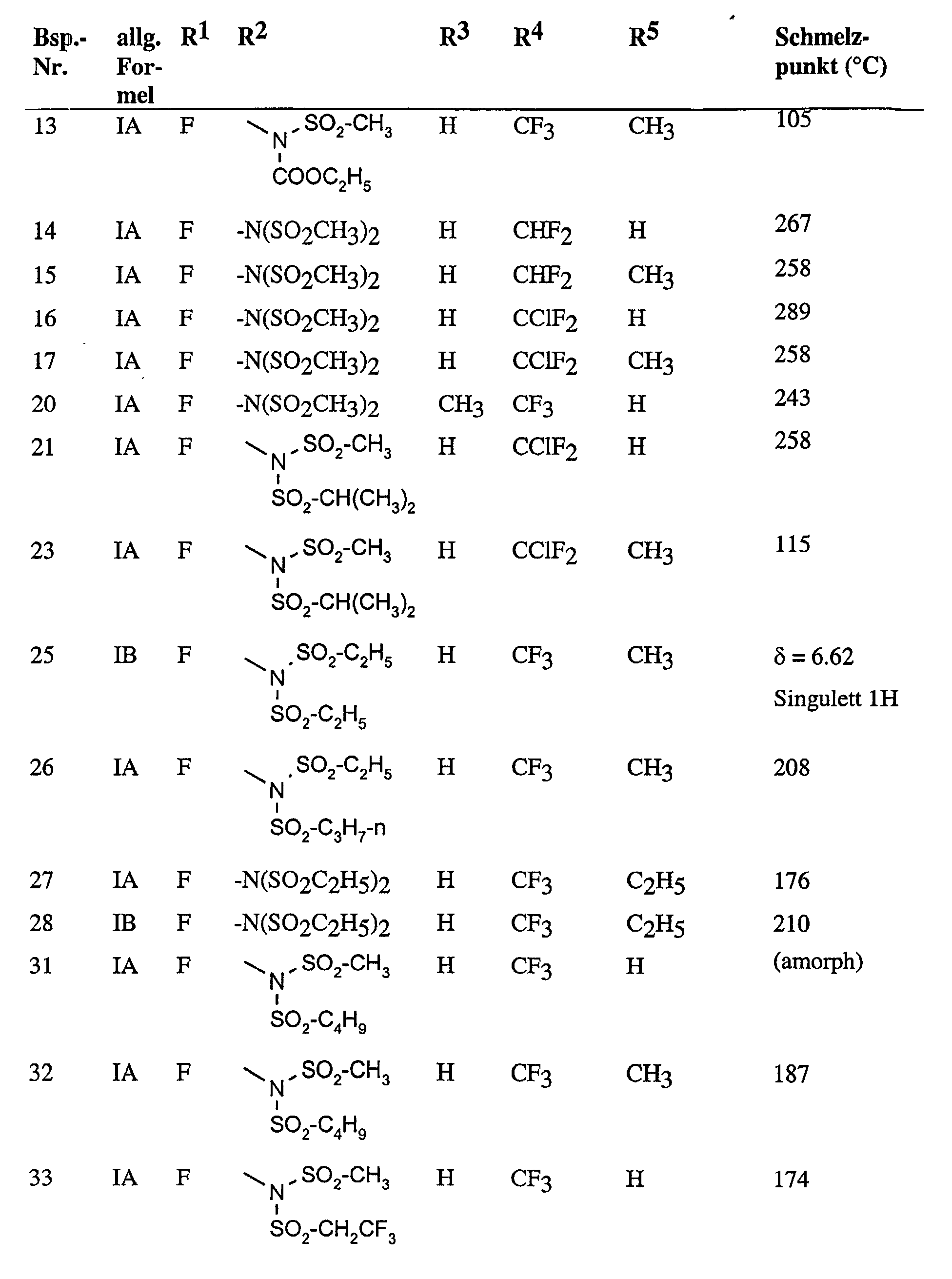

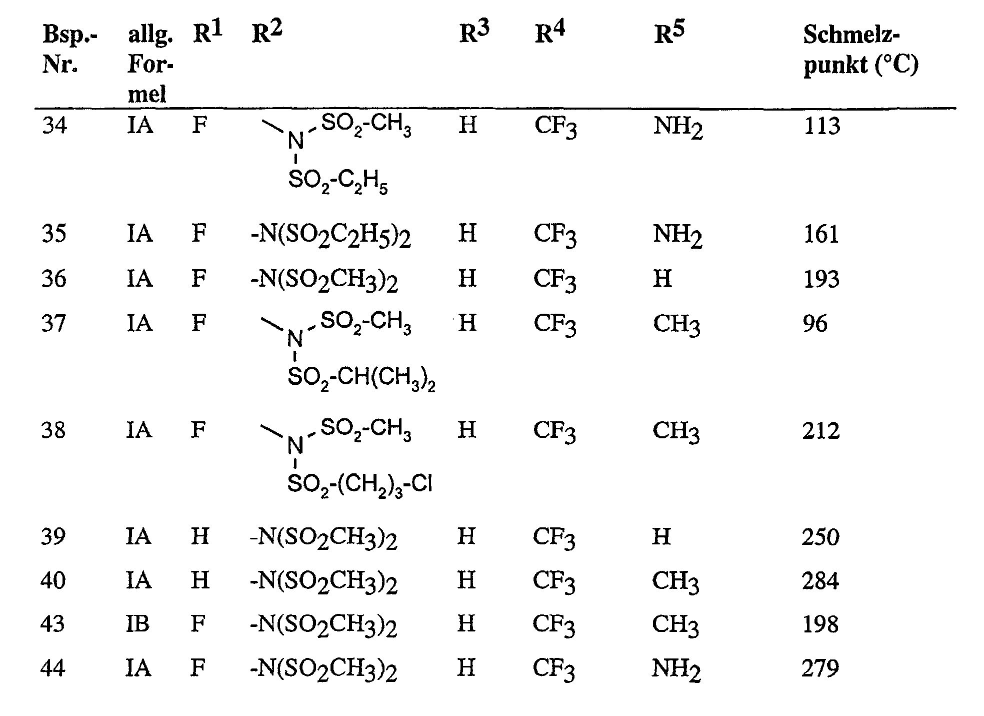

Die in Tabelle 2 als Beispiel 34 aufgeführte Verbindung kann beispielsweise wie folgt hergestellt werden: 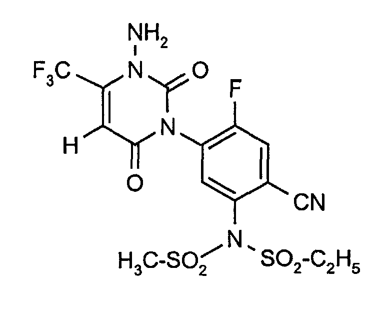 2,7 g (5,5 mMol) 1-[4-Cyano-2-fluor-5-(N-ethylsulfonyl-N-methylsulfonyl-amino)-phenyl]-3,6-dihydro-2,6-dioxo-4-trifluormethyl-1(2H)-pyrimidin werden in 10 g N,N-Dimethyl-formamid gelöst und zu dieser Lösung werden 0,3 g 60 %iges Natriumhydrid (7,5 mMol NaH) bei ca. 10°C gegeben. Die Mischung wird 15 Minuten gerührt und dann bei ca.10°C mit 1,1 g (5,5 mMol) 1-Aminooxy-2,4-dinitro-benzol in kleinen Portionen versetzt. Die Reaktionsmischung wird 3 Tage bei ca. 20°C gerührt. Dann wird mit 100 ml Essigsäureethylester verdünnt und die Mischung auf ca. 800 ml verdünnte wässrige Natriumchlorid-Lösung gegossen. Die wässrige Phase wird dreimal mit Essigsäureethylester extrahiert, die vereinigten organischen Extraktionslösungen mit Wasser gewaschen, mit Natriumsulfat getrocknet und filtriert. Das Filtrat wird eingeengt und der Rückstand durch Säulenchromatographie (Cyclohexan/Essigsäureethylester, Vol. 2/1) gereinigt.

Man erhält aus der Hauptfraktion 0,40 g (15% der Theorie) 1-[4-Cyano-2-fluor-5-(Nethylsulfonyl-N-methylsulfonyl-amino)-phenyl]-5-amino-3,6-dihydro-2,6-dioxo-4-trifluormethyl-1(2H)-pyrimidin vom Schmelzpunkt 113°C.

### Beispiele für die erfindungsgemäße Umwandlung:

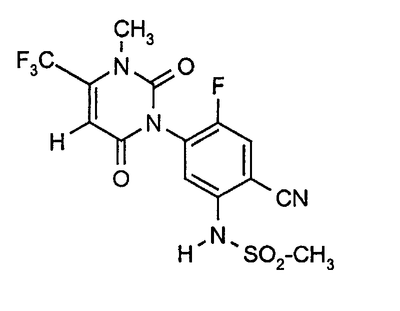

Eine Mischung aus 0,97 g (2 mMol) 1-[4-Cyano-2-fluor-5-(bis-methylsulfonyl-amino)-phenyl]-3,6-dihydro-2,6-dioxo-3-methyl-4-trifluormethyl-1(2H)-pyrimidin, 10 ml Wasser, 0,38 g (4 mMol) Natriumhydrogencarbonat und 20 ml Aceton wird 2 Tage bei 20°C gerührt. Dann wird das Aceton abdestilliert, die wässrige Lösung mit 2N-Salzsäure angesäuert und mit Essigsäureethylester geschüttelt. Die organische Phase wird mit Natriumsulfat getrocknet und filtriert. Das Filtrat wird eingeengt, der Rückstand in 1 ml Essigsäureethylester aufgenommen, mit Diethylether verdünnt und abgesaugt.

Man erhält 0,70 g (86 % der Theorie) 1-(4-Cyano-2-fluor-5-methylsulfonyl-aminophenyl)-3,6-dihydro-2,6-dioxo-3-methyl-4-trifluormethyl-1(2H)-pyrimidin vom Schmelzpunkt 192°C.

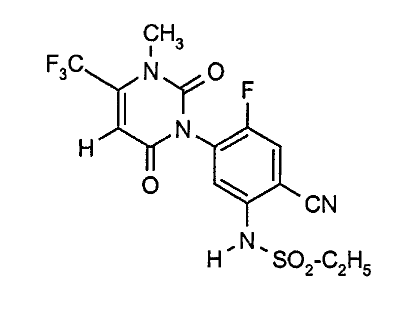

Eine Mischung aus 34 g (66 mMol) 1-[4-Cyano-2-fluor-5-(bis-ethylsulfonyl-amino)-phenyl]-3,6-dihydro-2,6-dioxo-3-methyl-4-trifluormethyl-1(2H)-pyrimidin, 250 ml Wasser, 11,1 g (130 mMol) Natriumhydrogencarbonat und 250 ml Aceton wird 8 Stunden bei 50°C gerührt. Anschließend wird das Aceton abdestilliert, der Rest des Reaktionsgemisches mit 500 ml Wasser verdünnt und filtriert. Das Filtrat wird dann mit 2N-Salzsäure angesäuert und abgesaugt. Das kristalline Produkt wird aus Diethylether/Essigsäureethylester (Vol.:95/5) umkristallisiert.

Man erhält 22 g (79 % der Theorie) 1-(4-Cyano-2-fluor-5-ethylsulfonyl-aminophenyl)-3,6-dihydro-2,6-dioxo-3-methyl-4-trifluormethyl-1(2H)-pyrimidin vom Schmelzpunkt 172°C.

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 2, 4, 6, 13 und 15 bei guter Verträglichkeit gegenüber Kulturpflanzen, wie z.B. Weizen, sehr starke Wirkung gegen Unkräuter.

### Beispiel B

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven Phaedon cochleariae besetzt, solange die Blätter noch feucht sind.

Nach der gewünschten Zeit wird die Abtötung in % bestimmt. Dabei bedeutet 100 %, dass alle Käfer-Larven abgetötet wurden; 0 % bedeutet, dass keine Käfer-Larven abgetötet wurden.

Bei diesem Test zeigt z.B. die Verbindung gemäß Herstellungsbeispiel 3 starke Wirksamkeit.

### Beispiel: C

### Plutella-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe Plutella maculipennis besetzt, solange die Blätter noch feucht sind.

Nach der gewünschten Zeit wird die Abtötung in % bestimmt. Dabei bedeutet 100 %, dass alle Raupen abgetötet wurden; 0 % bedeutet, dass keine Raupen abgetötet wurden.

Bei diesem Test zeigt z.B. die Verbindung gemäß Herstellungsbeispiel 3 starke Wirksamkeit.

### Beispiel D

### Spodoptera-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters Spodoptera frugiperda besetzt, solange die Blätter noch feucht sind.

Nach der gewünschten Zeit wird die Wirkung in % bestimmt. Dabei bedeutet 100 %, dass alle Raupen abgetötet wurden; 0 % bedeutet, dass keine Raupen abgetötet wurden.

Bei diesem Test zeigt z.B. die Verbindung gemäß Herstellungsbeispiel 3 starke Wirksamkeit.

## Patentansprüche

1. N-Cyanoaryl-Stickstoffheterocyclen der allgemeinen Formel (I) 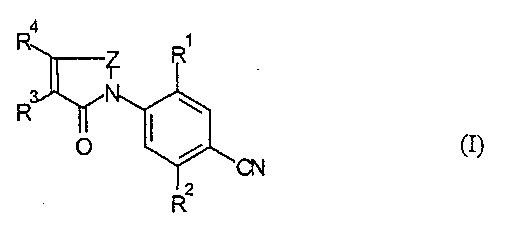 in welcher
R¹ für Wasserstoff, Fluor, Chlor oder Brom steht,
R² für die nachstehende Gruppierung steht, 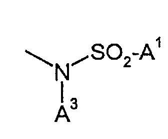 worin
A¹ für gegebenenfalls durch Halogen substituiertes Alkyl mit bis zu 10 Kohlenstoffatomen steht,
A³ für Alkylcarbonyl, Alkoxycarbonyl oder Alkylsulfonyl mit jeweils bis zu 6 Kohlenstoffatomen steht,
R³ für Wasserstoff, Halogen, Cyano oder für gegebenenfalls durch Halogen substituiertes Alkyl mit 1 bis 6 Kohlenstoffatomen steht,
R⁴ für gegebenenfalls durch Halogen oder C₁-C₄-Alkoxy substituiertes Alkyl mit 1 bis 6 Kohlenstoffatomen steht,
oder zusammen mit R³ für Alkandiyl mit 2 bis 8 Kohlenstoffatomen steht, und
Z für eine der nachstehenden Gruppierungen steht 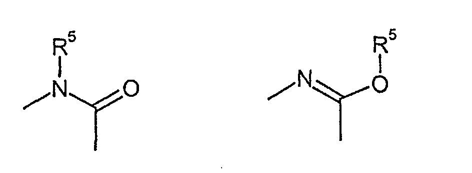 worin
R⁵ für Wasserstoff oder jeweils gegebenenfalls durch Fluor, Chlor, Brom, Cyano, C₁-C₄-Alkoxy, C₁-C₄-Alkyl-carbonyl oder C₁-C₄-Alkoxycarbonyl substituiertes Alkyl, Alkenyl, Alkinyl, Alkylcarbonyl oder Alkoxycarbonyl mit jeweils bis zu 6 Kohlenstoffatomen steht, oder für (jeweils nur an N gebundenes) Amino oder Hydroxy steht.

2. N-Cyanoaryl-Stickstoffheterocyclen gemäß Anspruch 1, **dadurch gekennzeichnet, dass**
R¹ für Wasserstoff, Fluor oder Chlor steht,
R² für die nachstehende Gruppierung steht, 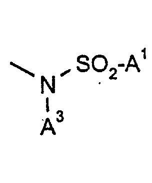 worin
A¹ für gegebenenfalls durch Fluor oder Chlor substituiertes Methyl, Ethyl, n- oder i-Propyl, n-, i-, s- oder t-Butyl, n-, i-, s- oder t-Pentyl steht,
A³ für Acetyl, Propionyl, n- oder i-Butyroyl, Methoxycarbonyl, Ethoxycarbonyl, n- oder i-Propoxycarbonyl, Methylsulfonyl, Ethylsulfonyl, n- oder i-Propylsulfonyl steht,
R³ für Wasserstoff, Fluor, Chlor, Brom, oder für jeweils gegebenenfalls durch Fluor und/oder Chlor substituiertes Methyl, Ethyl, n-oder i-Propyl steht,
R⁴ für jeweils gegebenenfalls durch Fluor und/oder Chlor substituiertes Methyl, Ethyl, n- oder i-Propyl steht,
oder zusammen mit R³ für Trimethylen oder Tetramethylen steht, und
Z für eine der nachstehenden Gruppierungen steht 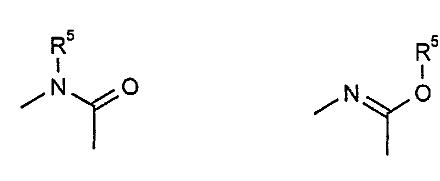 worin
R⁵ für Wasserstoff oder für jeweils gegebenenfalls durch Fluor, Chlor oder Cyano substituiertes Methyl, Ethyl, n- oder i-Propyl, n-, i- oder s-Butyl, Propenyl, Butenyl, Propinyl, Butinyl, Acetyl, Propionyl, Methoxycarbonyl oder Ethoxycarbonyl steht, oder für (jeweils nur an N gebundenes) Amino oder Hydroxy steht.

3. Verfahren zur Herstellung von N-Cyanoaryl-Stickstoffheterocyclen gemäß Anspruch 1 oder 2, **dadurch gekennzeichnet, dass** man
(a) zur Herstellung von Verbindungen der Formel (IA) und (IB), 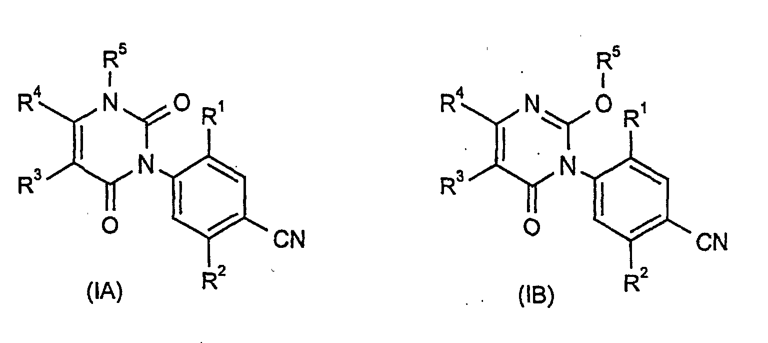 in welchen
R⁵ für Wasserstoff steht sowie R¹, R², R³ und R⁴ die in Anspruch 1 oder 2 angegebenen Bedeutungen haben,
Aminoalkensäureester der allgemeinen Formel (II) 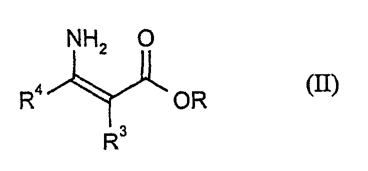 in welcher
R³ und R⁴ die in Anspruch 1 oder 2 angegebenen Bedeutungen haben und
R für Alkyl, Aryl oder Arylalkyl steht,
mit Cyanoarylisocyanaten der allgemeinen Formel (III) 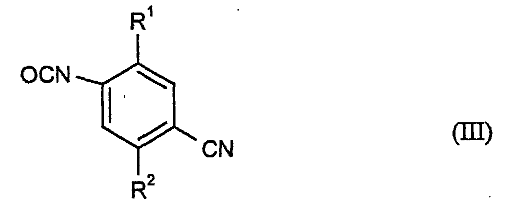 in welcher
R¹ und R² die oben angegebenen Bedeutungen haben,
gegebenenfalls in Gegenwart eines Reaktionshilfsmittels und gegebenenfalls in Gegenwart eines Verdünnungsmittels umsetzt,
oder dass man
(b) zur Herstellung von Verbindungen der Formeln (IA) und/oder (IB), in welchen R⁵ für jeweils gegebenenfalls durch Fluor, Chlor, Brom, Cyano, C₁-C₄-Alkoxy, C₁-C₄-Alkyl-carbonyl oder C₁-C₄-Alkoxycarbonyl substituiertes Alkyl, Alkenyl, Alkinyl, Alkylcarbonyl oder Alkoxycarbonyl mit jeweils bis zu 6 Kohlenstoffatomen steht sowie R¹, R², R³ und R⁴ die oben angegebenen Bedeutungen haben, N-Cyanoaryl-Stickstoffheterocyclen der allgemeinen Formeln (IA) und/oder (IB)
in welchen R⁵ für Wasserstoff steht sowie R¹, R², R³ und R⁴ die oben angegebenen Bedeutungen haben,
mit Alkylierungsmitteln bzw. Acylierungsmitteln der allgemeinen Formeln (V) oder (VI)
X¹-R⁵ (V)
R⁵-O-SO₂-O-R⁵ (VI)
in welchen
R⁵ die oben angegebene Bedeutung hat und
X¹ in der Formel (V) für Halogen steht,
gegebenenfalls in Gegenwart eines Säureakzeptors und gegebenenfalls in Gegenwart eines Verdünnungsmittels umsetzt,
oder dass man
(c) zur Herstellung von Verbindungen der Formel (I), in welcher R² für die nachstehende Gruppierung steht 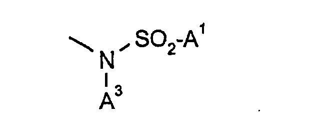 sowie A¹, A³, R¹, R³ R⁴ und Z die in Anspruch 1 oder 2 angegebenen Bedeutungen haben, N-Cyanoaryl-Stickstoffheterocyclen der allgemeinen Formel (I), in welcher R² für die Gruppierung -NH-SO₂-A¹ steht sowie A¹, R¹, R³, R⁴ und Z die oben angegebenen Bedeutungen haben,
mit Halogenverbindungen der allgemeinen Formel (VII)
X²-A³ (VII)
in welcher
A³ die oben angegebenen Bedeutungen hat und
X² für Halogen steht,
gegebenenfalls in Gegenwart eines Säureakzeptors und gegebenenfalls in Gegenwart eines Verdünnungsmittels umsetzt,
oder dass man
(d) zur Herstellung von Verbindungen der Formel (IA), in welcher R⁵ für Amino oder Hydroxy steht sowie R¹, R², R³ und R⁴ die oben angegebenen Bedeutungen haben,
N-Cyanoaryl-Stickstoffheterocyclen der allgemeinen Formeln (IA) und/oder (IB), in welchen R⁵ für Wasserstoff steht sowie R¹, R², R³ und R⁴ die oben angegebenen Bedeutungen haben,
mit elektrophilen Aminierungs- bzw. Hydroxylierungsmitteln gegebenenfalls in Gegenwart eines Säureakzeptors und gegebenenfalls in Gegenwart eines Verdünnungsmittels umsetzt.

4. Verwendung von substituierten N-Cyanoaryl-Stickstoffheterocyclen gemäß Anspruch 1 oder 2 zur Bekämpfung von unerwünschten Pflanzen.

5. Verwendung von substituierten N-Cyanoaryl-Stickstoffheterocyclen gemäß Anspruch 1 oder 2 zur Bekämpfung von unerwünschten Insekten.

6. Herbizide und insektizide Mittel, **gekennzeichnet durch** einen Gehalt an mindestens einem N-Cyanoaryl-Stickstoffheterocyclus gemäß Anspruch 1 oder 2.

## Claims

1. N-Cyanoaryl nitrogen heterocycles of the general formula (I) 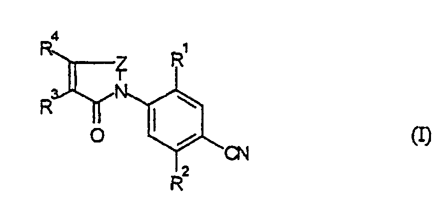 in which
R¹ represents hydrogen, fluorine, chlorine or bromine,
R² represents the group below 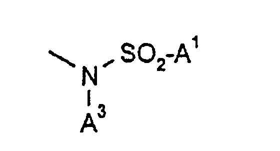 in which
A¹ represents alkyl which has up to 10 carbon atoms and is optionally substituted by halogen,
A³ represents alkylcarbonyl, alkoxycarbonyl or alkylsulphonyl, each of which has up to 6 carbon atoms,
R³ represents hydrogen, halogen, cyano, or alkyl having 1 to 6 carbon atoms which is optionally substituted by halogen,
R⁴ represents alkyl having 1 to 6 carbon atoms which is optionally substituted by halogen or C₁-C₄-alkoxy,
or together with R³ represents alkanediyl having 2 to 8 carbon atoms, and
Z represents one of the following groups 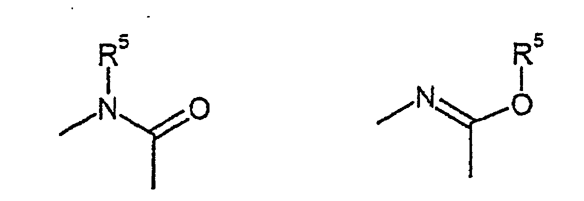 where
R⁵ represents hydrogen, or represents alkyl, alkenyl, alkinyl, alkylcarbonyl or alkoxycarbonyl, each of which has up to 6 carbon atoms and each of which is optionally substituted by fluorine, chlorine, bromine, cyano, C₁-C₄-alkoxy, C₁-C₄-alkyl-carbonyl or C₁-C₄-alkoxycarbonyl or represents amino or hydroxyl (each of which is only attached to N).

2. N-Cyanoaryl nitrogen heterocycles according to Claim 1, **characterized in that**
R¹ represents hydrogen, fluorine or chlorine,
R² represents the group below 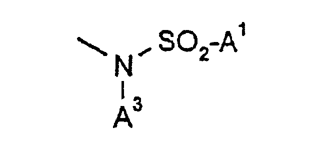 where
A¹ represents methyl, ethyl, n- or i-propyl, n-, i-, s- or t-butyl, n-, i-, s- or t-pentyl, which is optionally substituted by fluorine or chlorine,
A³ xrepresents acetyl, propionyl, n- or i-butyroyl, methoxycarbonyl, ethoxycarbonyl, n- or i-propoxycarbonyl, methylsulphonyl, ethylsulphonyl, n- or i-propylsulphonyl,
R³ xrepresents hydrogen, fluorine, chlorine, bromine, or represents methyl, ethyl, nor i-propyl, each of which is optionally substituted by fluorine and/or chlorine,
R⁴ represents methyl, ethyl, n- or i-propyl, each of which is optionally substituted by fluorine and/or chlorine,
or together with R³ represents trimethylene or tetramethylene, and
Z represents one of the groups below 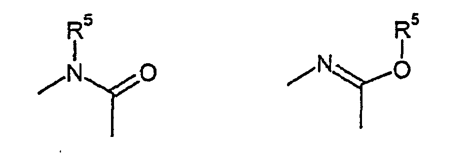 where
R⁵ xrepresents hydrogen, or represents methyl, ethyl, n- or i-propyl, n-, i- or s-butyl, propenyl, butenyl, propinyl, butinyl, acetyl, propionyl, methoxycarbonyl or ethoxycarbonyl, each of which is optionally substituted by fluorine, chlorine or cyano, or represents amino or hydroxyl (each of which is only attached to N).

3. Process for the preparation of N-cyanoaryl nitrogen heterocycles according to Claim 1 or 2, **characterized in that**
(a) to prepare compounds of the formulae (IA) and (IB) 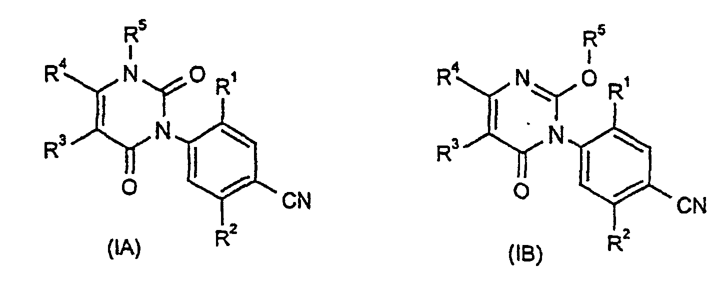 in which
R⁵ represents hydrogen and R¹, R², R³ and R⁴ have the meanings given in Claim 1 or 2,
aminoalkenoic esters of the general formula (II) 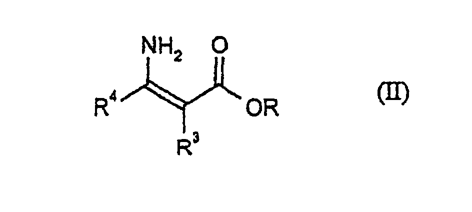 in which
R³ and R⁴ have the meanings given in Claim 1 or 2 and
R represents alkyl, aryl or arylalkyl,
are reacted with cyanoaryl isocyanates of the general formula (III) 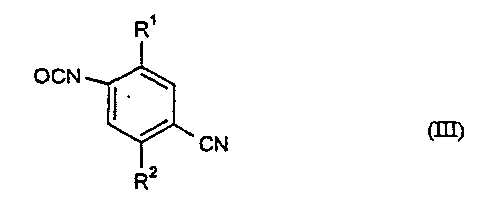 in which
R¹ and R² have the abovementioned meanings,
if appropriate in the presence of a reaction auxiliary and if appropriate in the presence of a diluent,
or **in that**
(b)to prepare compounds of the formulae (IA) and/or (IB) in which R⁵ represents alkyl, alkenyl, alkinyl, alkylcarbonyl or alkoxycarbonyl, each of which has up to 6 carbon atoms and each of which is optionally substituted by fluorine, chlorine, bromine, cyano, C₁-C₄-alkoxy, C₁-C₄-alkylcarbonyl or C₁-C₄-alkoxycarbonyl, and R¹, R², R³ and R⁴ have the abovementioned meanings,
N-cyanoaryl nitrogen heterocycles of the general formulae (IA) and/or (IB)
in which R⁵ represents hydrogen and R¹, R², R³ and R⁴ have the abovementioned meanings,
are reacted with alkylating agents or acylating agents of the general formulae (V) or (VI)
X¹-R⁵ (V)
R⁵-O-SO₂-O-R⁵ (VI)
in which
R⁵ has the abovementioned meaning and
X¹ in formula (V) represents halogen,
if appropriate in the presence of an acid acceptor and if appropriate in the presence of a diluent,
or **in that**
(c) to prepare compounds of the formula (I) in which R² represents the following group 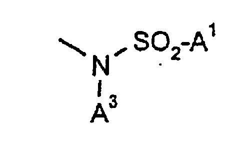 and A¹, A³, R¹, R³, R⁴ and Z have the meanings given in Claim 1 or 2,
N-cyanoaryl nitrogen heterocycles of the general formula (I) in which R² represents the group -NH-SO₂-A¹ and A¹, R¹, R³, R⁴ and Z have the abovementioned meanings,
are reacted with halogen compounds of the general formula (VII)
X²-A³ (VII)
in which
A³ has the abovementioned meanings and
X² represents halogen,
if appropriate in the presence of an acid acceptor and if appropriate in the presence of a diluent,
or **in that**
(d) to prepare compounds of the formula (IA) in which R⁵ represents amino or hydroxyl and R¹, R², R³, and R⁴ have the abovementioned meanings,
N-cyanoaryl nitrogen heterocycles of the general formulae (IA) and/or (IB) in which R⁵ represents hydrogen and R¹, R², R³ and R⁴ have the abovementioned meanings,
are reacted with electrophilic aminating or hydroxylating agents, if appropriate in the presence of an acid acceptor and if appropriate in the presence of a diluent.

4. Use of substituted N-cyanoaryl nitrogen heterocycles according to Claim 1 or 2 for combating undesirable plants.

5. Use of substituted N-cyanoaryl nitrogen heterocycles according to Claim 1 or 2 for combating undesirable insects.

6. Herbicidal and insecticidal compositions, **characterized in that** they comprise at least one N-cyanoaryl nitrogen heterocycle according to Claim 1 or 2.

## Revendications

1. Hétérocycles azotés N-cyanoaryle de la formule générale (I) : 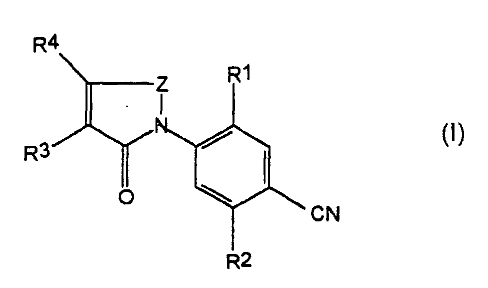 dans laquelle :
R¹ représente l'atome d'hydrogène, de fluor, de chlore ou de brome ;
R² représente le groupement suivant : 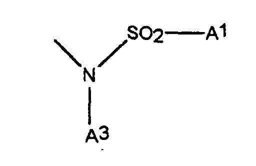
où :
A¹ représente un radical alcoyle le cas échéant substitué par un atome d'halogène, ayant jusqu'à 10 atomes de carbone ;
A³ représente un radical alcoylcarbonyle, alcoxycarbonyle ou alcoylsulfonyle avec chaque fois jusqu'à 6 atomes de carbone ;
R³ représente l'atome d'hydrogène, un atome d'halogène, le radical cyano ou un radical alcoyle le cas échéant substitué par un atome d'halogène, ayant 1 à 6 atomes de carbone ;
R⁴ représente radical alcoyle ayant 1 à 6 atomes de carbone, le cas échéant substitué par un atome d'halogène ou un radical alcoxy en C₁-C₄,
ou avec R³ représente un radical alcanediyle ayant 2 à 8 atomes de carbone, et
Z représente l'un des groupements suivants : 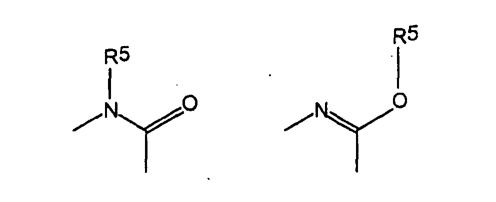 où :
R⁵ représente l'atome d'hydrogène ou un radical alcoyle, alcényle, alcynyle, alcoylcarbonyle ou alcoxycarbonyle, ayant chaque fois jusqu'à 6 atomes de carbone, chaque fois le cas échéant substitué par l'atome de fluor, de chlore, de brome, le radical cyano, un radical alcoxy en C₁-C₄, (alcoyle en C₁-C₄)carbonyle ou (alcoxy en C₁-C₄)carbonyle, ou représente le radical amino ou hydroxyle (chaque fois relié seulement sur le N).

2. Hétérocycles azotés N-cyanoaryle suivant la revendication 1, **caractérisés en ce que** :
R¹ représente l'atome d'hydrogène, de fluor ou de chlore ;
R² représente le groupement suivant : 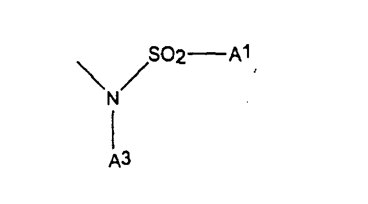 où :
A¹ représente le radical méthyle, éthyle, n- ou i-propyle, n-, i-, s- ou t-butyle, n-, i-, s- ou t-pentyle, le cas échéant substitué par l'atome de fluor ou de chlore ;
A³ représente le radical acétyle, propionyle, n- ou i-butyroyle, méthoxycarbonyle, éthoxycarbonyle, n- ou i-propoxycarbonyle, méthylsulfonyle, éthylsulfonyle, n- ou i-propylsulfonyle ;
R³ représente l'atome d'hydrogène, de fluor, de chlore, de brome ou le radical méthyle, éthyle, n- ou i-propyle le cas échéant substitué par l'atome de fluor et/ou de chlore ;
R⁴ représente le radical méthyle, éthyle, n- ou i-propyle le cas échéant substitué par l'atome de fluor et/ou de chlore,
ou avec R³, représente le radical triméthylène ou tétraméthylène, et
Z représente l'un des groupements suivants : 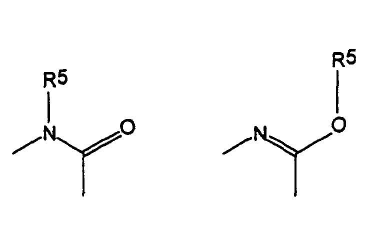 où :
R⁵ représente l'atome d'hydrogène ou le radical méthyle, éthyle, n- ou i-propyle, n-, i- ou s-butyle, propényle, butényle, propynyle, butynyle, acetyle, propionyle, méthoxycarbonyle ou éthoxycarbonyle, chaque fois le cas échéant substitué par l'atome de fluor, de chlore ou le radical cyano, représente le radical amino
ou hydroxyle (chaque fois relié seulement sur le N).

3. Procédé de préparation d'hétérocycles azotés N-cyanoaryle suivant la revendication 1 ou 2, **caractérisé en ce que**
(a) pour la préparation des composés des formules (IA) et (IB) : 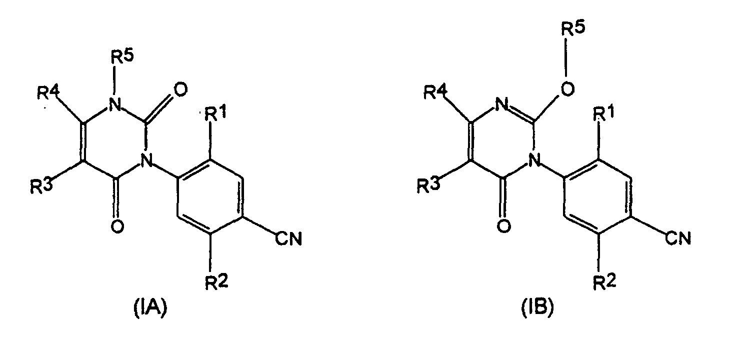 dans lesquelles :
R⁵ représente l'atome d'hydrogène, alors que R¹, R², R³ et R⁴ ont les significations indiquées à la revendication 1 ou 2,
on fait réagir des esters d'acide aminoalcènoïque de la formule générale (II) : 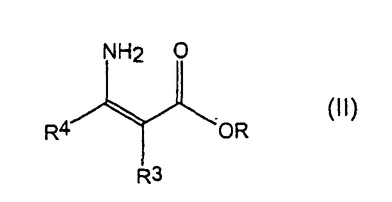
dans laquelle :
R³ et R⁴ ont les significations indiquées à la revendication 1 ou 2, et
R représente un radical alcoyle, aryle ou arylalcoyle,
avec des isocyanates de cyanoaryle de la formule générale (III) : 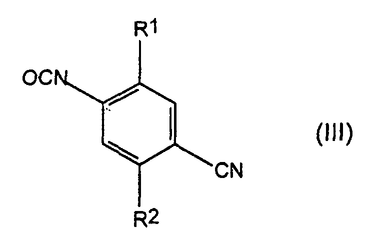
dans laquelle :
R¹ et R² ont les significations indiquées ci-dessus,
le cas échéant en présence d'un auxiliaire de réaction et le cas échéant, en présence d'un agent de dilution,
ou **en ce que**
(b) pour la préparation des composés des formules (IA) et/ou (IB), dans lesquelles R⁵ représente un radical alcoyle, alcényle, alcynyle, alcoylcarbonyle ou alcoxycarbonyle, ayant chaque fois jusqu'à 6 atomes de carbone, chaque fois le cas échéant substitué par l'atome de fluor, de chlore, de brome, le radical cyano, un radical alcoxy en C₁-C₄, (alcoyle en C₁-C₄)carbonyle ou (alcoxy en C₁-C₄)carbonyle, alors que R¹, R², R³ et R⁴ ont les significations indiquées ci-dessus, on fait réagir des hétérocycles azotés N-cyanoaryle des formules générales (IA) et/ou (IB), dans lesquelles R⁵ représenté l'atome d'hydrogène, alors que R¹, R², R³ et R⁴ ont les significations indiquées ci-dessus,
avec des agents d'alcoylation ou agents d'acylation des formules générales (V) ou (VI) :
X¹-R⁵ (V)
R⁵-O-SO₂-O-R⁵ (VI)
dans lesquelles:
R⁵ a la signification indiquée ci-dessus, et
X¹ dans la formule (V), représente un atome d'halogène,
le cas échéant en présence d'un accepteur d'acide et le cas échéant, en présence d'un agent de dilution,
ou **en ce que**
(c) pour la préparation de composés de la formule (I), dans laquelle R² représente le groupement suivant : 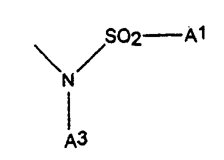 alors que A¹, A³, R¹, R³, R⁴ et Z ont les significations indiquées à la revendication 1 ou 2, on fait réagir des hétérocycles azotés N-cyanoaryle de la formule générale (I), dans laquelle R² représente le groupement -NH-SO₂-A¹, alors que A¹, R¹, R³, R⁴ et Z ont les significations indiquées ci-dessus,
avec des composés halogénés de la formule générale (VII) :
X²-A³ (VII)
dans laquelle:
A³ a la signification indiquée ci-dessus, et
X² représente un atome d'halogène,
le cas échéant en présence d'un accepteur d'acide et le cas échéant, en présence d'un agent de dilution,
ou **en ce que**
(d) pour la préparation de composés de la formule (IA), dans laquelle R⁵ représente le radical amino ou hydroxyle alors que R¹, R², R³ et R⁴ ont les significations indiquées ci-dessus, on fait réagir des hétérocycles azotés N-cyanoaryle des formules générales (IA) et/ou (IB), dans lesquelles R⁵ représente l'atome d'hydrogène, alors que R¹, R², R³ et R⁴ ont les significations indiquées ci-dessus,
avec des agents électrophiles d'amination ou d'hydroxylation, le cas échéant en présence d'un accepteur d'acide et le cas échéant, en présence d'un. agent de dilution.

4. Utilisation d'hétérocycles azotés N-cyanoaryle substitués selon la revendication 1 ou 2, pour lutter contre des végétaux non souhaités.

5. Utilisation d'hétérocycles azotés N-cyanoaryle substitués selon la revendication 1 ou 2, pour lutter contré des insectes non désirés.

6. Agent herbicide et insecticide, **caractérisé par** une teneur en au moins un hétérocycle azoté N-cyanoaryle selon la revendication 1 ou 2.
